# EUROPÄISCHE PATENTSCHRIFT

(11) **EP 1 841 859 B1**
(45) Veröffentlichungstag und Bekanntmachung des Hinweises auf die Patenterteilung: **02.07.2014**
(21) Anmeldenummer: 05823727.2
(22) Anmeldetag: 29.12.2005
(51) Int. Cl.: C12N 9/02, C12P 13/08, C12N 1/20

(54) **ALLELE DES MQO-GENS AUS CORYNEFORMEN BAKTERIEN**
ALLELES OF THE MQO-GENE FROM CORYNEFORM BACTERIA
ALLELES DU GENE MQO ISSUS DE BACTERIES CORYNEFORMES

(30) Priorität: 19.01.2005 DE 102005002489; 12.07.2005 DE 102005032429
(43) Veröffentlichungstag der Anmeldung: 10.10.2007
(73) Patentinhaber: Evonik Degussa GmbH, 45128 Essen (DE)
(72) Erfinder: BATHE, Brigitte, 33154 Salzkotten (DE); HANS, Stephan, 49078 Osnabrück (DE); SCHISCHKA, Natalie, 33649 Salzkotten (DE); THIERBACH, Georg, 33613 Salzkotten (DE)
(74) Vertreter: Ioannidis, Johannes
(86) Internationale Anmeldenummer: PCT/EP2005/057216
(87) Internationale Veröffentlichungsnummer: WO 2006/077004

(56) Entgegenhaltungen:
- EP-A- 1 038 969
- WO-A-02/086137
- PFEFFERLE W ET AL: "BIOTECHNOLOGICAL MANUFACTURE OF LYSINE" ADVANCES IN BIOCHEMICAL ENGINEERING, BIOTECHNOLOGY, SPRINGER, BERLIN, DE, vol. 79, 2003, pages 59-112, XP008032623 ISSN: 0724-6145

### SEQUENCE LISTING

<110> Degussa AG
<120> Allele des mqo-Gens aus coryneformen Bakterien
<130> 040041 BT
<160> 26
<170> PatentIn version 3.3
<210> 1
   <211> 1503
   <212> DNA
   <213> Corynebacterium glutamicum
<220>
   <221> CDS
   <222> (1)..(1500)
   <223> mqo-wildtypgen
<400> 1
   atg tca gat tcc ccg aag aac gca ccg agg att acc gat gag gca gat        48
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg        96
   Val Val Leu Ile Gly Ala Gly Ile Met Ser Ser Thr Leu Gly Ala Met
               20                  25                  30
   ctg cgt cag ctg gag cca agc tgg act cag atc gtc ttc gag cgt ttg       144
   Leu Arg Gln Leu Glu Pro Ser Trp Thr Gln Ile Val Phe Glu Arg Leu
           35                  40                  45
   gat gga ccg gca caa gag tcg tcc tcc ccg tgg aac aat gca gga acc       192
   Asp Gly Pro Ala Gln Glu Ser Ser Ser Pro Trp Asn Asn Ala Gly Thr
       50                  55                  60
   ggc cac tct gct cta tgc gag ctg aac tac acc cca gag gtt aag ggc       240
   Gly His Ser Ala Leu Cys Glu Leu Asn Tyr Thr Pro Glu Val Lys Gly
   65                  70                  75                  80
   aag gtt gaa att gcc aag gct gta gga atc aac gag aag ttc cag gtt       288
   Lys Val Glu Ile Ala Lys Ala Val Gly Ile Asn Glu Lys Phe Gln Val
                   85                  90                  95
   tcc cgt cag ttc tgg tct cac ctc gtt gaa gag gga gtg ctg tct gat       336
   Ser Arg Gln Phe Trp Ser His Leu Val Glu Glu Gly Val Leu Ser Asp
               100                 105                 110
   cct aag gaa ttc atc aac cct gtt cct cac gta tct ttc ggc cag ggc       384
   Pro Lys Glu Phe Ile Asn Pro Val Pro His Val Ser Phe Gly Gln Gly
           115                 120                 125
   gca gat cag gtt gca tac atc aag gct cgc tac gaa gct ttg aag gat       432
   Ala Asp Gln Val Ala Tyr Ile Lys Ala Arg Tyr Glu Ala Leu Lys Asp
       130                 135                 140
   cac cca ctc ttc cag ggc atg acc tac gct gac gat gaa gct acc ttc       480
   His Pro Leu Phe Gln Gly Met Thr Tyr Ala Asp Asp Glu Ala Thr Phe
   145                 150                 155                 160
   acc gag aag ctg cct ttg atg gca aag ggc cgt gac ttc tct gat cca       528
   Thr Glu Lys Leu Pro Leu Met Ala Lys Gly Arg Asp Phe Ser Asp Pro
                   165                 170                 175
   gta gca atc tct tgg atc gat gaa ggc acc gac atc aac tac ggt gct       576
   Val Ala Ile Ser Trp Ile Asp Glu Gly Thr Asp Ile Asn Tyr Gly Ala
               180                 185                 190
   cag acc aag cag tac ctg gat gca gct gaa gtt gaa ggc act gaa atc      624
   Gln Thr Lys Gln Tyr Leu Asp Ala Ala Glu Val Glu Gly Thr Glu Ile
           195                 200                 205
   cgc tat ggc cac gaa gtc aag agc atc aag gct gat ggc gca aag tgg      672
   Arg Tyr Gly His Glu Val Lys Ser Ile Lys Ala Asp Gly Ala Lys Trp
       210                 215                 220
   atc gtg acc gtc aag aac gta cac act ggc gac acc aag acc atc aag      720
   Ile Val Thr Val Lys Asn Val His Thr Gly Asp Thr Lys Thr Ile Lys
   225                 230                 235                 240
   gca aac ttc gtg ttc gtc ggc gca ggc gga tac gca ctg gat ctg ctt      768
   Ala Asn Phe Val Phe Val Gly Ala Gly Gly Tyr Ala Leu Asp Leu Leu
                   245                 250                 255
   cgc agc gca ggc atc cca cag gtc aag ggc ttc gct gga ttc cca gta      816
   Arg Ser Ala Gly Ile Pro Gln Val Lys Gly Phe Ala Gly Phe Pro Val
               260                 265                 270
   tcc ggc ctg tgg ctt cgt tgc acc aac gag gaa ctg atc gag cag cac      864
   Ser Gly Leu Trp Leu Arg Cys Thr Asn Glu Glu Leu Ile Glu Gln His
           275                 280                 285
   gca gcc aag gta tat ggc aag gca tct gtt ggc gct cct cca atg tct      912
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc      960
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc     1008
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac     1056
   Tyr Leu Asp Leu Phe Lys Ser Ile Arg Pro Asp Asn Ile Pro Ser Tyr
               340                 345                 350
   ctt ggc gtt gct gct cag gaa ttt gat ctg acc aag tac ctt gtc act     1104
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac     1152
   Glu Val Leu Lys Asp Gln Asp Lys Arg Met Asp Ala Leu Arg Glu Tyr
       370                 375                 380
   atg cca gag gca caa aac ggc gat tgg gag acc atc gtt gcc gga cag     1200
   Met Pro Glu Ala Gln Asn Gly Asp Trp Glu Thr Ile Val Ala Gly Gln
   385                 390                 395                 400
   cgt gtt cag gtt att aag cct gca gga ttc cct aag ttc ggt tcc ctg     1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga     1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc     1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac     1392
   Glu Leu Leu Glu Arg Cys Phe Gly Asp Arg Met Ile Glu Trp Gly Asp
       450                     455                     460
   aag ctg aag gac atg atc cct tcc tac ggc aag aag ctt gct tcc gag      1440
   Lys Leu Lys Asp Met Ile Pro Ser Tyr Gly Lys Lys Leu Ala Ser Glu
   465                     470                     475                     480
   cca gca ctg ttt gag cag cag tgg gca cgc acc cag aag acc ctg aag      1488
   Pro Ala Leu Phe Glu Gln Gln Trp Ala Arg Thr Gln Lys Thr Leu Lys
                   485                     490                     495
   ctt gag gaa gcc taa                                                  1503
   Leu Glu Glu Ala
               500
<210> 2
   <211> 500
   <212> PRT
   <213> Corynebacterium glutamicum
<400> 2
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
           115                 120                 125
   Ala Asp Gln Val Ala Tyr Ile Lys Ala Arg Tyr Glu Ala Leu Lys Asp
       130                 135                 140
   His Pro Leu Phe Gln Gly Met Thr Tyr Ala Asp Asp Glu Ala Thr Phe
   145                 150                 155                 160
   Thr Glu Lys Leu Pro Leu Met Ala Lys Gly Arg Asp Phe Ser Asp Pro
                   165                 170                 175
   Val Ala Ile Ser Trp Ile Asp Glu Gly Thr Asp Ile Asn Tyr Gly Ala
               180                 185                 190
   Gln Thr Lys Gln Tyr Leu Asp Ala Ala Glu Val Glu Gly Thr Glu Ile
           195                 200                 205
   Arg Tyr Gly His Glu Val Lys Ser Ile Lys Ala Asp Gly Ala Lys Trp
       210                 215                 220
   Ile Val Thr Val Lys Asn Val His Thr Gly Asp Thr Lys Thr Ile Lys
   225                 230                 235                 240
   Ala Asn Phe Val Phe Val Gly Ala Gly Gly Tyr Ala Leu Asp Leu Leu
                   245                 250                 255
   Arg Ser Ala Gly Ile Pro Gln Val Lys Gly Phe Ala Gly Phe Pro Val
               260                 265                 270
   Ser Gly Leu Trp Leu Arg Cys Thr Asn Glu Glu Leu Ile Glu Gln His
           275                 280                 285
   Ala Ala Lys Val Tyr Gly Lys Ala Ser Val Gly Ala Pro Pro Met Ser
       290                 295                 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
               340                 345                 350
   Leu Gly Val Ala Ala Gln Glu Phe Asp Leu Thr Lys Tyr Leu Val Thr
           355                 360                 365
   Glu Val Leu Lys Asp Gln Asp Lys Arg Met Asp Ala Leu Arg Glu Tyr
       370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465 470 475 480
   Pro Ala Leu Phe Glu Gln Gln Trp Ala Arg Thr Gln Lys Thr Leu Lys
   485 490 495
   Leu Glu Glu Ala
   500
<210> 3
   <211> 2002
   <212> DNA
   <213> Corynebacterium glutamicum
<220>
   <221> CDS
   <222> (350)..(1849)
   <223> mqo wildtyp-Gen
<400> 3
   agtcgactga aatgttcacg tggtgaaact tccgcgatac tactcatgtt tgcgaattgc      60
   acatttacta actttgcaaa ttgggggagg gggtagcgcg ggggaggaat tcgcatgaga     120
   aaggggaata tcccgtgctt gtttattcag ctcgaggtgg caggcgtaca ctctatattc     180
   acggacaatg tgtacccacg ctttcttgta agaaacaaga agggtaacgc cccacgcgtc     240
   agtcaaaaat atggccaaca cttgcattcg ggtgctggcg atcatttatg agatgacgcc     300
   ttgtgttggt gttcggcaga gaactcgcgg agataaaagg aagttgaac atg tca gat     358
                                                        Met Ser Asp
                                                        1
   tcc ccg aag aac gca ccg agg att acc gat gag gca gat gta gtt ctc       406
   Ser Pro Lys Asn Ala Pro Arg Ile Thr Asp Glu Ala Asp Val Val Leu
       5                   10                  15
   att ggt gcc ggt atc atg agc tcc acg ctg ggt gca atg ctg cgt cag       454
   Ile Gly Ala Gly Ile Met Ser Ser Thr Leu Gly Ala Met Leu Arg Gln
   20                  25                  30                  35
   ctg gag cca agc tgg act cag atc gtc ttc gag cgt ttg gat gga ccg       502
   Leu Glu Pro Ser Trp Thr Gln Ile Val Phe Glu Arg Leu Asp Gly Pro
                   40                  45                  50
   gca caa gag tcg tcc tcc ccg tgg aac aat gca gga acc ggc cac tct       550
   Ala Gln Glu Ser Ser Ser Pro Trp Asn Asn Ala Gly Thr Gly His Ser
               55                  60                  65
   gct cta tgc gag ctg aac tac acc cca gag gtt aag ggc aag gtt gaa       598
   Ala Leu Cys Glu Leu Asn Tyr Thr Pro Glu Val Lys Gly Lys Val Glu
           70                  75                  80
   att gcc aag gct gta gga atc aac gag aag ttc cag gtt tcc cgt cag       646
   Ile Ala Lys Ala Val Gly Ile Asn Glu Lys Phe Gln Val Ser Arg Gln
       85                  90                  95
   ttc tgg tct cac ctc gtt gaa gag gga gtg ctg tct gat cct aag gaa       694
   Phe Trp Ser His Leu Val Glu Glu Gly Val Leu Ser Asp Pro Lys Glu
   100                 105                 110                 115
   ttc atc aac cct gtt cct cac gta tct ttc ggc cag ggc gca gat cag       742
   Phe Ile Asn Pro Val Pro His Val Ser Phe Gly Gln Gly Ala Asp Gln
                   120                 125                 130
   gtt gca tac atc aag gct cgc tac gaa gct ttg aag gat cac cca ctc 790
   Val Ala Tyr Ile Lys Ala Arg Tyr Glu Ala Leu Lys Asp His Pro Leu
   135 140 145
   ttc cag ggc atg acc tac gct gac gat gaa gct acc ttc acc gag aag 838
   Phe Gln Gly Met Thr Tyr Ala Asp Asp Glu Ala Thr Phe Thr Glu Lys
   150 155 160
   ctg cct ttg atg gca aag ggc cgt gac ttc tct gat cca gta gca atc 886
   Leu Pro Leu Met Ala Lys Gly Arg Asp Phe Ser Asp Pro Val Ala Ile
   165 170 175
   tct tgg atc gat gaa ggc acc gac atc aac tac ggt gct cag acc aag 934
   Ser Trp Ile Asp Glu Gly Thr Asp Ile Asn Tyr Gly Ala Gln Thr Lys
   180 185 190 195
   cag tac ctg gat gca gct gaa gtt gaa ggc act gaa atc cgc tat ggc 982
   Gln Tyr Leu Asp Ala Ala Glu Val Glu Gly Thr Glu Ile Arg Tyr Gly
   200 205 210
   cac gaa gtc aag agc atc aag gct gat ggc gca aag tgg atc gtg acc 1030
   His Glu Val Lys Ser Ile Lys Ala Asp Gly Ala Lys Trp Ile Val Thr
   215 220 225
   gtc aag aac gta cac act ggc gac acc aag acc atc aag gca aac ttc 1078
   Val Lys Asn Val His Thr Gly Asp Thr Lys Thr Ile Lys Ala Asn Phe
   230 235 240
   gtg ttc gtc ggc gca ggc gga tac gca ctg gat ctg ctt cgc agc gca 1126
   Val Phe Val Gly Ala Gly Gly Tyr Ala Leu Asp Leu Leu Arg Ser Ala
   245 250 255
   ggc atc cca cag gtc aag ggc ttc gct gga ttc cca gta tcc ggc ctg 1174
   Gly Ile Pro Gln Val Lys Gly Phe Ala Gly Phe Pro Val Ser Gly Leu
   260 265 270 275
   tgg ctt cgt tgc acc aac gag gaa ctg atc gag cag cac gca gcc aag 1222
   Trp Leu Arg Cys Thr Asn Glu Glu Leu Ile Glu Gln His Ala Ala Lys
   280 285 290
   gta tat ggc aag gca tct gtt ggc gct cct cca atg tct gtt cct cac 1270
   Val Tyr Gly Lys Ala Ser Val Gly Ala Pro Pro Met Ser Val Pro His
   295 300 305
   ctt gac acc cgc gtt atc gag ggt gaa aag ggt ctg ctc ttt gga cct 1318
   Leu Asp Thr Arg Val Ile Glu Gly Glu Lys Gly Leu Leu Phe Gly Pro
   310 315 320
   tac ggt ggc tgg acc cct aag ttc ttg aag gaa ggc tcc tac ctg gac 1366
   Tyr Gly Gly Trp Thr Pro Lys Phe Leu Lys Glu Gly Ser Tyr Leu Asp
   325 330 335
   ctg ttc aag tcc atc cgc cca gac aac att cct tcc tac ctt ggc gtt 1414
   Leu Phe Lys Ser Ile Arg Pro Asp Asn Ile Pro Ser Tyr Leu Gly Val
   340 345 350 355
   gct gct cag gaa ttt gat ctg acc aag tac ctt gtc act gaa gtt ctc 1462
   Ala Ala Gln Glu Phe Asp Leu Thr Lys Tyr Leu Val Thr Glu Val Leu
   360 365 370
   aag gac cag gac aag cgt atg gat gct ctt cgc gag tac atg cca gag 1510
   Lys Asp Gln Asp Lys Arg Met Asp Ala Leu Arg Glu Tyr Met Pro Glu
   375 380 385
   gca caa aac ggc gat tgg gag acc atc gtt gcc gga cag cgt gtt cag 1558
   Ala Gln Asn Gly Asp Trp Glu Thr Ile Val Ala Gly Gln Arg Val Gln
   390 395 400
   gtt att aag cct gca gga ttc cct aag ttc ggt tcc ctg gaa ttc ggc      1606
   Val Ile Lys Pro Ala Gly Phe Pro Lys Phe Gly Ser Leu Glu Phe Gly
       405                 410                 415
   acc acc ttg atc aac aac tcc gaa ggc acc atc gcc gga ttg ctc ggt      1654
   Thr Thr Leu Ile Asn Asn Ser Glu Gly Thr Ile Ala Gly Leu Leu Gly
   420                 425                 430                 435
   gct tcc cct gga gca tcc atc gca cct tcc gca atg atc gag ctg ctt      1702
   Ala Ser Pro Gly Ala Ser Ile Ala Pro Ser Ala Met Ile Glu Leu Leu
                   440                 445                 450
   gag cgt tgc ttc ggt gac cgc atg atc gag tgg ggc gac aag ctg aag      1750
   Glu Arg Cys Phe Gly Asp Arg Met Ile Glu Trp Gly Asp Lys Leu Lys
               455                 460                 465
   gac atg atc cct tcc tac ggc aag aag ctt gct tcc gag cca gca ctg      1798
   Asp Met Ile Pro Ser Tyr Gly Lys Lys Leu Ala Ser Glu Pro Ala Leu
           470                 475                 480
   ttt gag cag cag tgg gca cgc acc cag aag acc ctg aag ctt gag gaa      1846
   Phe Glu Gln Gln Trp Ala Arg Thr Gln Lys Thr Leu Lys Leu Glu Glu
       485                 490                 495
   gcc taaatcttct aactgctttc tttaaagcac ccgcacatgt ctgttgaggt           1899
   Ala
   500
   ttcacctgcg gagacaatct ccgccttcat gggttggaac tgacacagtt gaaggcatgt    1959
   cgggtgcttt gcgtattctt tgccagtgtg atttaggcga cac                      2002
<210> 4
   <211> 500
   <212> PRT
   <213> Corynebactenum glutamicum
<400> 4
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
   115 120 125
   Ala Asp Gln Val Ala Tyr Ile Lys Ala Arg Tyr Glu Ala Leu Lys Asp
   130 135 140
   His Pro Leu Phe Gln Gly Met Thr Tyr Ala Asp Asp Glu Ala Thr Phe
   145 150 155 160
   Thr Glu Lys Leu Pro Leu Met Ala Lys Gly Arg Asp Phe Ser Asp Pro
   165 170 175
   Val Ala Ile Ser Trp Ile Asp Glu Gly Thr Asp Ile Asn Tyr Gly Ala
   180 185 190
   Gln Thr Lys Gln Tyr Leu Asp Ala Ala Glu Val Glu Gly Thr Glu Ile
   195 200 205
   Arg Tyr Gly His Glu Val Lys Ser Ile Lys Ala Asp Gly Ala Lys Trp
   210 215 220
   Ile Val Thr Val Lys Asn Val His Thr Gly Asp Thr Lys Thr Ile Lys
   225 230 235 240
   Ala Asn Phe Val Phe Val Gly Ala Gly Gly Tyr Ala Leu Asp Leu Leu
   245 250 255
   Arg Ser Ala Gly Ile Pro Gln Val Lys Gly Phe Ala Gly Phe Pro Val
   260 265 270
   Ser Gly Leu Trp Leu Arg Cys Thr Asn Glu Glu Leu Ile Glu Gln His
   275 280 285
   Ala Ala Lys Val Tyr Gly Lys Ala Ser Val Gly Ala Pro Pro Met Ser
   290 295 300
   Val Pro His Leu Asp Thr Arg Val Ile Glu Gly Glu Lys Gly Leu Leu
   305 310 315 320
   Phe Gly Pro Tyr Gly Gly Trp Thr Pro Lys Phe Leu Lys Glu Gly Ser
   325 330 335
   Tyr Leu Asp Leu Phe Lys Ser Ile Arg Pro Asp Asn Ile Pro Ser Tyr
   340 345 350
   Leu Gly Val Ala Ala Gln Glu Phe Asp Leu Thr Lys Tyr Leu Val Thr
   355 360 365
   Glu Val Leu Lys Asp Gln Asp Lys Arg Met Asp Ala Leu Arg Glu Tyr
   370 375 380
   Met Pro Glu Ala Gln Asn Gly Asp Trp Glu Thr Ile Val Ala Gly Gln
   385 390 395 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
                   485                 490                 495
   Leu Glu Glu Ala
               500
<210> 5
   <211> 1503
   <212> DNA
   <213> Corynebacterium glutamicum
<220>
   <221> CDS
   <222> (1)..(1500)
   <223> mqo-Allel
<220>
   <221> mutation
   <222> (332)..(332)
   <223> Transition: Austausch von cytosin gegen Thymin
<400> 5
   atg tca gat tcc ccg aag aac gca ccg agg att acc gat gag gca gat        48
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg        96
   Val Val Leu Ile Gly Ala Gly Ile Met Ser Ser Thr Leu Gly Ala Met
               20                  25                  30
   ctg cgt cag ctg gag cca agc tgg act cag atc gtc ttc gag cgt ttg       144
   Leu Arg Gln Leu Glu Pro Ser Trp Thr Gln Ile Val Phe Glu Arg Leu
           35                  40                  45
   gat gga ccg gca caa gag tcg tcc tcc ccg tgg aac aat gca gga acc       192
   Asp Gly Pro Ala Gln Glu Ser Ser Ser Pro Trp Asn Asn Ala Gly Thr
       50                  55                  60
   ggc cac tct gct cta tgc gag ctg aac tac acc cca gag gtt aag ggc       240
   Gly His Ser Ala Leu Cys Glu Leu Asn Tyr Thr Pro Glu Val Lys Gly
   65                  70                  75                  80
   aag gtt gaa att gcc aag gct gta gga atc aac gag aag ttc cag gtt       288
   Lys Val Glu Ile Ala Lys Ala Val Gly Ile Asn Glu Lys Phe Gln Val
                   85                      90                      95
   tcc cgt cag ttc tgg tct cac ctc gtt gaa gag gga gtg ctg ttt gat      336
   Ser Arg Gln Phe Trp Ser His Leu Val Glu Glu Gly Val Leu Phe Asp
               100                     105                     110
   cct aag gaa ttc atc aac cct gtt cct cac gta tct ttc ggc cag ggc      384
   Pro Lys Glu Phe Ile Asn Pro Val Pro His Val Ser Phe Gly Gln Gly
           115                     120                     125
   gca gat cag gtt gca tac atc aag gct cgc tac gaa gct ttg aag gat      432
   Ala Asp Gln Val Ala Tyr Ile Lys Ala Arg Tyr Glu Ala Leu Lys Asp
       130                     135                     140
   cac cca ctc ttc cag ggc atg acc tac gct gac gat gaa gct acc ttc      480
   His Pro Leu Phe Gln Gly Met Thr Tyr Ala Asp Asp Glu Ala Thr Phe
   145                     150                     155                     160
   acc gag aag ctg cct ttg atg gca aag ggc cgt gac ttc tct gat cca      528
   Thr Glu Lys Leu Pro Leu Met Ala Lys Gly Arg Asp Phe Ser Asp Pro
                   165                     170                     175
   gta gca atc tct tgg atc gat gaa ggc acc gac atc aac tac ggt gct      576
   Val Ala Ile Ser Trp Ile Asp Glu Gly Thr Asp Ile Asn Tyr Gly Ala
               180                     185                     190
   cag acc aag cag tac ctg gat gca gct gaa gtt gaa ggc act gaa atc      624
   Gln Thr Lys Gln Tyr Leu Asp Ala Ala Glu Val Glu Gly Thr Glu Ile
           195                     200                     205
   cgc tat ggc cac gaa gtc aag agc atc aag gct gat ggc gca aag tgg      672
   Arg Tyr Gly His Glu Val Lys Ser Ile Lys Ala Asp Gly Ala Lys Trp
       210                     215                     220
   atc gtg acc gtc aag aac gta cac act ggc gac acc aag acc atc aag      720
   Ile Val Thr Val Lys Asn Val His Thr Gly Asp Thr Lys Thr Ile Lys
   225                     230                     235                     240
   gca aac ttc gtg ttc gtc ggc gca ggc gga tac gca ctg gat ctg ctt      768
   Ala Asn Phe Val Phe Val Gly Ala Gly Gly Tyr Ala Leu Asp Leu Leu
                   245                     250                     255
   cgc agc gca ggc atc cca cag gtc aag ggc ttc gct gga ttc cca gta      816
   Arg Ser Ala Gly Ile Pro Gln Val Lys Gly Phe Ala Gly Phe Pro Val
               260                     265                     270
   tcc ggc ctg tgg ctt cgt tgc acc aac gag gaa ctg atc gag cag cac      864
   Ser Gly Leu Trp Leu Arg Cys Thr Asn Glu Glu Leu Ile Glu Gln His
           275                     280                     285
   gca gcc aag gta tat ggc aag gca tct gtt ggc gct cct cca atg tct      912
   Ala Ala Lys Val Tyr Gly Lys Ala Ser Val Gly Ala Pro Pro Met Ser
       290                     295                     300
   gtt cct cac ctt gac acc cgc gtt atc gag ggt gaa aag ggt ctg ctc      960
   Val Pro His Leu Asp Thr Arg Val Ile Glu Gly Glu Lys Gly Leu Leu
   305                     310                     315                     320
   ttt gga cct tac ggt ggc tgg acc cct aag ttc ttg aag gaa ggc tcc     1008
   Phe Gly Pro Tyr Gly Gly Trp Thr Pro Lys Phe Leu Lys Glu Gly Ser
                   325                     330                     335
   tac ctg gac ctg ttc aag tcc atc cgc cca gac aac att cct tcc tac     1056
   Tyr Leu Asp Leu Phe Lys Ser Ile Arg Pro Asp Asn Ile Pro Ser Tyr
               340                     345                     350
   ctt ggc gtt gct gct cag gaa ttt gat ctg acc aag tac ctt gtc act     1104
   Leu Gly Val Ala Ala Gln Glu Phe Asp Leu Thr Lys Tyr Leu Val Thr
               355                    360                    365
   gaa gtt ctc aag gac cag gac aag cgt atg gat gct ctt cgc gag tac      1152
   Glu Val Leu Lys Asp Gln Asp Lys Arg Met Asp Ala Leu Arg Glu Tyr
       370                 375                 380
   atg cca gag gca caa aac ggc gat tgg gag acc atc gtt gcc gga cag      1200
   Met Pro Glu Ala Gln Asn Gly Asp Trp Glu Thr Ile Val Ala Gly Gln
   385                 390                 395                 400
   cgt gtt cag gtt att aag cct gca gga ttc cct aag ttc ggt tcc ctg      1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga      1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc      1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac      1392
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag      1440
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ctg aag      1488
   Pro Ala Leu Phe Glu Gln Gln Trp Ala Arg Thr Gln Lys Thr Leu Lys
                   485                 490                 495
   ctt gag gaa gcc taa                                                  1503
   Leu Glu Glu Ala
               500
<210> 6
   <211> 500
   <212> PRT
   <213> Corynebactenum glutamicum
<400> 6
   Met Ser Asp Ser Pro Lys Asn Ala Pro Arg Ile Thr Asp Glu Ala Asp
   1 5 10 15
   Val Val Leu Ile Gly Ala Gly Ile Met Ser Ser Thr Leu Gly Ala Met
   20 25 30
   Leu Arg Gln Leu Glu Pro Ser Trp Thr Gln Ile Val Phe Glu Arg Leu
   35 40 45
   Asp Gly Pro Ala Gln Glu Ser Ser Ser Pro Trp Asn Asn Ala Gly Thr
   50 55 60
   Gly His Ser Ala Leu Cys Glu Leu Asn Tyr Thr Pro Glu Val Lys Gly
   65 70 75 80
   Lys Val Glu Ile Ala Lys Ala Val Gly Ile Asn Glu Lys Phe Gln Val
   85 90 95
   Ser Arg Gln Phe Trp Ser His Leu Val Glu Glu Gly Val Leu Phe Asp
   100 105 110
   Pro Lys Glu Phe Ile Asn Pro Val Pro His Val Ser Phe Gly Gln Gly
   115 120 125
   Ala Asp Gln Val Ala Tyr Ile Lys Ala Arg Tyr Glu Ala Leu Lys Asp
   130 135 140
   His Pro Leu Phe Gln Gly Met Thr Tyr Ala Asp Asp Glu Ala Thr Phe
   145 150 155 160
   Thr Glu Lys Leu Pro Leu Met Ala Lys Gly Arg Asp Phe Ser Asp Pro
   165 170 175
   Val Ala Ile Ser Trp Ile Asp Glu Gly Thr Asp Ile Asn Tyr Gly Ala
   180 185 190
   Gln Thr Lys Gln Tyr Leu Asp Ala Ala Glu Val Glu Gly Thr Glu Ile
   195 200 205
   Arg Tyr Gly His Glu Val Lys Ser Ile Lys Ala Asp Gly Ala Lys Trp
   210 215 220
   Ile Val Thr Val Lys Asn Val His Thr Gly Asp Thr Lys Thr Ile Lys
   225 230 235 240
   Ala Asn Phe Val Phe Val Gly Ala Gly Gly Tyr Ala Leu Asp Leu Leu
   245 250 255
   Arg Ser Ala Gly Ile Pro Gln Val Lys Gly Phe Ala Gly Phe Pro Val
   260 265 270
   Ser Gly Leu Trp Leu Arg Cys Thr Asn Glu Glu Leu Ile Glu Gln His
   275 280 285
   Ala Ala Lys Val Tyr Gly Lys Ala Ser Val Gly Ala Pro Pro Met Ser
   290 295 300
   Val Pro His Leu Asp Thr Arg Val Ile Glu Gly Glu Lys Gly Leu Leu
   305 310 315 320
   Phe Gly Pro Tyr Gly Gly Trp Thr Pro Lys Phe Leu Lys Glu Gly Ser
   325 330 335
   Tyr Leu Asp Leu Phe Lys Ser Ile Arg Pro Asp Asn Ile Pro Ser Tyr
   340 345 350
   Leu Gly Val Ala Ala Gln Glu Phe Asp Leu Thr Lys Tyr Leu Val Thr
   355 360 365
   Glu Val Leu Lys Asp Gln Asp Lys Arg Met Asp Ala Leu Arg Glu Tyr
       370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
                   485                 490                 495
   Leu Glu Glu Ala
               500
<210> 7
   <211> 1503
   <212> DNA
   <213> Corynebacterium glutamicum
<220>
   <221> CDS
   <222> (1)..(1500)
   <223> mqo-Allel
<220>
   <221> mutation
   <222> (331)..(331)
   <223> Transversion: Austausch von Thymin gegen Guanin
<400> 7
   atg tca gat tcc ccg aag aac gca ccg agg att acc gat gag gca gat      48
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg      96
   Val Val Leu Ile Gly Ala Gly Ile Met Ser Ser Thr Leu Gly Ala Met
               20                  25                  30
   ctg cgt cag ctg gag cca agc tgg act cag atc gtc ttc gag cgt ttg      144
   Leu Arg Gln Leu Glu Pro Ser Trp Thr Gln Ile Val Phe Glu Arg Leu
           35                  40                  45
   gat gga ccg gca caa gag tcg tcc tcc ccg tgg aac aat gca gga acc      192
   Asp Gly Pro Ala Gln Glu Ser Ser Ser Pro Trp Asn Asn Ala Gly Thr
       50                  55                  60
   ggc cac tct gct cta tgc gag ctg aac tac acc cca gag gtt aag ggc      240
   Gly His Ser Ala Leu Cys Glu Leu Asn Tyr Thr Pro Glu Val Lys Gly
   65                  70                  75                  80
   aag gtt gaa att gcc aag gct gta gga atc aac gag aag ttc cag gtt      288
   Lys Val Glu Ile Ala Lys Ala Val Gly Ile Asn Glu Lys Phe Gln Val
                   85                  90                  95
   tcc cgt cag ttc tgg tct cac ctc gtt gaa gag gga gtg ctg gct gat      336
   Ser Arg Gln Phe Trp Ser His Leu Val Glu Glu Gly Val Leu Ala Asp
               100                 105                 110
   cct aag gaa ttc atc aac cct gtt cct cac gta tct ttc ggc cag ggc      384
   Pro Lys Glu Phe Ile Asn Pro Val Pro His Val Ser Phe Gly Gln Gly
           115                 120                 125
   gca gat cag gtt gca tac atc aag gct cgc tac gaa gct ttg aag gat      432
   Ala Asp Gln Val Ala Tyr Ile Lys Ala Arg Tyr Glu Ala Leu Lys Asp
       130                 135                 140
   cac cca ctc ttc cag ggc atg acc tac gct gac gat gaa gct acc ttc      480
   His Pro Leu Phe Gln Gly Met Thr Tyr Ala Asp Asp Glu Ala Thr Phe
   145                 150                 155                 160
   acc gag aag ctg cct ttg atg gca aag ggc cgt gac ttc tct gat cca      528
   Thr Glu Lys Leu Pro Leu Met Ala Lys Gly Arg Asp Phe Ser Asp Pro
                   165                 170                 175
   gta gca atc tct tgg atc gat gaa ggc acc gac atc aac tac ggt gct      576
   Val Ala Ile Ser Trp Ile Asp Glu Gly Thr Asp Ile Asn Tyr Gly Ala
               180                 185                 190
   cag acc aag cag tac ctg gat gca gct gaa gtt gaa ggc act gaa atc      624
   Gln Thr Lys Gln Tyr Leu Asp Ala Ala Glu Val Glu Gly Thr Glu Ile
           195                 200                 205
   cgc tat ggc cac gaa gtc aag agc atc aag gct gat ggc gca aag tgg      672
   Arg Tyr Gly His Glu Val Lys Ser Ile Lys Ala Asp Gly Ala Lys Trp
       210                 215                 220
   atc gtg acc gtc aag aac gta cac act ggc gac acc aag acc atc aag      720
   Ile Val Thr Val Lys Asn Val His Thr Gly Asp Thr Lys Thr Ile Lys
   225                 230                 235                 240
   gca aac ttc gtg ttc gtc ggc gca ggc gga tac gca ctg gat ctg ctt      768
   Ala Asn Phe Val Phe Val Gly Ala Gly Gly Tyr Ala Leu Asp Leu Leu
                   245                 250                 255
   cgc agc gca ggc atc cca cag gtc aag ggc ttc gct gga ttc cca gta      816
   Arg Ser Ala Gly Ile Pro Gln Val Lys Gly Phe Ala Gly Phe Pro Val
               260                 265                 270
   tcc ggc ctg tgg ctt cgt tgc acc aac gag gaa ctg atc gag cag cac      864
   Ser Gly Leu Trp Leu Arg Cys Thr Asn Glu Glu Leu Ile Glu Gln His
           275                 280                 285
   gca gcc aag gta tat ggc aag gca tct gtt ggc gct cct cca atg tct      912
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc      960
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc     1008
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac      1056
   Tyr Leu Asp Leu Phe Lys Ser Ile Arg Pro Asp Asn Ile Pro Ser Tyr
               340                 345                 350
   ctt ggc gtt gct gct cag gaa ttt gat ctg acc aag tac ctt gtc act      1104
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac      1152
   Glu Val Leu Lys Asp Gln Asp Lys Arg Met Asp Ala Leu Arg Glu Tyr
       370                 375                 380
   atg cca gag gca caa aac ggc gat tgg gag acc atc gtt gcc gga cag      1200
   Met Pro Glu Ala Gln Asn Gly Asp Trp Glu Thr Ile Val Ala Gly Gln
   385                 390                 395                 400
   cgt gtt cag gtt att aag cct gca gga ttc cct aag ttc ggt tcc ctg      1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga      1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc      1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac      1392
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag      1440
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ctg aag      1488
   Pro Ala Leu Phe Glu Gln Gln Trp Ala Arg Thr Gln Lys Thr Leu Lys
                   485                 490                 495
   ctt gag gaa gcc taa                                                  1503
   Leu Glu Glu Ala
               500
<210> 8
   <211> 500
   <212> PRT
   <213> Corynebacterium glutamicum
<400> 8
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ala Asp
               100                 105                 110
   Pro Lys Glu Phe Ile Asn Pro Val Pro His Val Ser Phe Gly Gln Gly
           115                 120                 125
   Ala Asp Gln Val Ala Tyr Ile Lys Ala Arg Tyr Glu Ala Leu Lys Asp
       130                 135                 140
   His Pro Leu Phe Gln Gly Met Thr Tyr Ala Asp Asp Glu Ala Thr Phe
   145                 150                 155                 160
   Thr Glu Lys Leu Pro Leu Met Ala Lys Gly Arg Asp Phe Ser Asp Pro
                   165                 170                 175
   Val Ala Ile Ser Trp Ile Asp Glu Gly Thr Asp Ile Asn Tyr Gly Ala
               180                 185                 190
   Gln Thr Lys Gln Tyr Leu Asp Ala Ala Glu Val Glu Gly Thr Glu Ile
           195                 200                 205
   Arg Tyr Gly His Glu Val Lys Ser Ile Lys Ala Asp Gly Ala Lys Trp
       210                 215                 220
   Ile Val Thr Val Lys Asn Val His Thr Gly Asp Thr Lys Thr Ile Lys
   225                 230                 235                 240
   Ala Asn Phe Val Phe Val Gly Ala Gly Gly Tyr Ala Leu Asp Leu Leu
                   245                 250                 255
   Arg Ser Ala Gly Ile Pro Gln Val Lys Gly Phe Ala Gly Phe Pro Val
               260                 265                 270
   Ser Gly Leu Trp Leu Arg Cys Thr Asn Glu Glu Leu Ile Glu Gln His
           275                 280                 285
   Ala Ala Lys Val Tyr Gly Lys Ala Ser Val Gly Ala Pro Pro Met Ser
       290                 295                 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
   340 345 350
   Leu Gly Val Ala Ala Gln Glu Phe Asp Leu Thr Lys Tyr Leu Val Thr
   355 360 365
   Glu Val Leu Lys Asp Gln Asp Lys Arg Met Asp Ala Leu Arg Glu Tyr
   370 375 380
   Met Pro Glu Ala Gln Asn Gly Asp Trp Glu Thr Ile Val Ala Gly Gln
   385 390 395 400
   Arg Val Gln Val Ile Lys Pro Ala Gly Phe Pro Lys Phe Gly Ser Leu
   405 410 415
   Glu Phe Gly Thr Thr Leu Ile Asn Asn Ser Glu Gly Thr Ile Ala Gly
   420 425 430
   Leu Leu Gly Ala Ser Pro Gly Ala Ser Ile Ala Pro Ser Ala Met Ile
   435 440 445
   Glu Leu Leu Glu Arg Cys Phe Gly Asp Arg Met Ile Glu Trp Gly Asp
   450 455 460
   Lys Leu Lys Asp Met Ile Pro Ser Tyr Gly Lys Lys Leu Ala Ser Glu
   465 470 475 480
   Pro Ala Leu Phe Glu Gln Gln Trp Ala Arg Thr Gln Lys Thr Leu Lys
   485 490 495
   Leu Glu Glu Ala
   500
<210> 9
   <211> 1503
   <212> DNA
   <213> Corynebacterium glutamicum
<220>
   <221> CDS
   <222> (1)..(1500)
   <223> mqo-Allel
<220>
   <221> mutation
   <222> (331)..(331)
   <223> Transversion: Austausch von Thymin gegen Guanin
<220>
   <221> mutation
   <222> (601)..(601)
   <223> Transversion: Austausch von Guanin gegen Thymin
<400> 9
   atg tca gat tcc ccg aag aac gca ccg agg att acc gat gag gca gat         48
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg 96
   Val Val Leu Ile Gly Ala Gly Ile Met Ser Ser Thr Leu Gly Ala Met
   20 25 30
   ctg cgt cag ctg gag cca agc tgg act cag atc gtc ttc gag cgt ttg 144
   Leu Arg Gln Leu Glu Pro Ser Trp Thr Gln Ile Val Phe Glu Arg Leu
   35 40 45
   gat gga ccg gca caa gag tcg tcc tcc ccg tgg aac aat gca gga acc 192
   Asp Gly Pro Ala Gln Glu Ser Ser Ser Pro Trp Asn Asn Ala Gly Thr
   50 55 60
   ggc cac tct gct cta tgc gag ctg aac tac acc cca gag gtt aag ggc 240
   Gly His Ser Ala Leu Cys Glu Leu Asn Tyr Thr Pro Glu Val Lys Gly
   65 70 75 80
   aag gtt gaa att gcc aag gct gta gga atc aac gag aag ttc cag gtt 288
   Lys Val Glu Ile Ala Lys Ala Val Gly Ile Asn Glu Lys Phe Gln Val
   85 90 95
   tcc cgt cag ttc tgg tct cac ctc gtt gaa gag gga gtg ctg gct gat 336
   Ser Arg Gln Phe Trp Ser His Leu Val Glu Glu Gly Val Leu Ala Asp
   100 105 110
   cct aag gaa ttc atc aac cct gtt cct cac gta tct ttc ggc cag ggc 384
   Pro Lys Glu Phe Ile Asn Pro Val Pro His Val Ser Phe Gly Gln Gly
   115 120 125
   gca gat cag gtt gca tac atc aag gct cgc tac gaa gct ttg aag gat 432
   Ala Asp Gln Val Ala Tyr Ile Lys Ala Arg Tyr Glu Ala Leu Lys Asp
   130 135 140
   cac cca ctc ttc cag ggc atg acc tac gct gac gat gaa gct acc ttc 480
   His Pro Leu Phe Gln Gly Met Thr Tyr Ala Asp Asp Glu Ala Thr Phe
   145 150 155 160
   acc gag aag ctg cct ttg atg gca aag ggc cgt gac ttc tct gat cca 528
   Thr Glu Lys Leu Pro Leu Met Ala Lys Gly Arg Asp Phe Ser Asp Pro
   165 170 175
   gta gca atc tct tgg atc gat gaa ggc acc gac atc aac tac ggt gct 576
   Val Ala Ile Ser Trp Ile Asp Glu Gly Thr Asp Ile Asn Tyr Gly Ala
   180 185 190
   cag acc aag cag tac ctg gat gca tct gaa gtt gaa ggc act gaa atc 624
   Gln Thr Lys Gln Tyr Leu Asp Ala Ser Glu Val Glu Gly Thr Glu Ile
   195 200 205
   cgc tat ggc cac gaa gtc aag agc atc aag gct gat ggc gca aag tgg 672
   Arg Tyr Gly His Glu Val Lys Ser Ile Lys Ala Asp Gly Ala Lys Trp
   210 215 220
   atc gtg acc gtc aag aac gta cac act ggc gac acc aag acc atc aag 720
   Ile Val Thr Val Lys Asn Val His Thr Gly Asp Thr Lys Thr Ile Lys
   225 230 235 240
   gca aac ttc gtg ttc gtc ggc gca ggc gga tac gca ctg gat ctg ctt 768
   Ala Asn Phe Val Phe Val Gly Ala Gly Gly Tyr Ala Leu Asp Leu Leu
   245 250 255
   cgc agc gca ggc atc cca cag gtc aag ggc ttc gct gga ttc cca gta 816
   Arg Ser Ala Gly Ile Pro Gln Val Lys Gly Phe Ala Gly Phe Pro Val
   260 265 270
   tcc ggc ctg tgg ctt cgt tgc acc aac gag gaa ctg atc gag cag cac 864
   Ser Gly Leu Trp Leu Arg Cys Thr Asn Glu Glu Leu Ile Glu Gln His
   275 280 285
   gca gcc aag gta tat ggc aag gca tct gtt ggc gct cct cca atg tct        912
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc        960
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc       1008
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac       1056
   Tyr Leu Asp Leu Phe Lys Ser Ile Arg Pro Asp Asn Ile Pro Ser Tyr
               340                 345                 350
   ctt ggc gtt gct gct cag gaa ttt gat ctg acc aag tac ctt gtc act       1104
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac       1152
   Glu Val Leu Lys Asp Gln Asp Lys Arg Met Asp Ala Leu Arg Glu Tyr
       370                 375                 380
   atg cca gag gca caa aac ggc gat tgg gag acc atc gtt gcc gga cag       1200
   Met Pro Glu Ala Gln Asn Gly Asp Trp Glu Thr Ile Val Ala Gly Gln
   385                 390                 395                 400
   cgt gtt cag gtt att aag cct gca gga ttc cct aag ttc ggt tcc ctg       1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga       1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc       1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac       1392
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag       1440
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ctg aag       1488
   Pro Ala Leu Phe Glu Gln Gln Trp Ala Arg Thr Gln Lys Thr Leu Lys
                   485                 490                 495
   ctt gag gaa gcc taa                                                   1503
   Leu Glu Glu Ala
               500
<210> 10
   <211> 500
   <212> PRT
   <213> Corynebacterium glutamicum
<400> 10
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
   20 25 30
   Leu Arg Gln Leu Glu Pro Ser Trp Thr Gln Ile Val Phe Glu Arg Leu
   35 40 45
   Asp Gly Pro Ala Gln Glu Ser Ser Ser Pro Trp Asn Asn Ala Gly Thr
   50 55 60
   Gly His Ser Ala Leu Cys Glu Leu Asn Tyr Thr Pro Glu Val Lys Gly
   65 70 75 80
   Lys Val Glu Ile Ala Lys Ala Val Gly Ile Asn Glu Lys Phe Gln Val
   85 90 95
   Ser Arg Gln Phe Trp Ser His Leu Val Glu Glu Gly Val Leu Ala Asp
   100 105 110
   Pro Lys Glu Phe Ile Asn Pro Val Pro His Val Ser Phe Gly Gln Gly
   115 120 125
   Ala Asp Gln Val Ala Tyr Ile Lys Ala Arg Tyr Glu Ala Leu Lys Asp
   130 135 140
   His Pro Leu Phe Gln Gly Met Thr Tyr Ala Asp Asp Glu Ala Thr Phe
   145 150 155 160
   Thr Glu Lys Leu Pro Leu Met Ala Lys Gly Arg Asp Phe Ser Asp Pro
   165 170 175
   Val Ala Ile Ser Trp Ile Asp Glu Gly Thr Asp Ile Asn Tyr Gly Ala
   180 185 190
   Gln Thr Lys Gln Tyr Leu Asp Ala Ser Glu Val Glu Gly Thr Glu Ile
   195 200 205
   Arg Tyr Gly His Glu Val Lys Ser Ile Lys Ala Asp Gly Ala Lys Trp
   210 215 220
   Ile Val Thr Val Lys Asn Val His Thr Gly Asp Thr Lys Thr Ile Lys
   225 230 235 240
   Ala Asn Phe Val Phe Val Gly Ala Gly Gly Tyr Ala Leu Asp Leu Leu
   245 250 255
   Arg Ser Ala Gly Ile Pro Gln Val Lys Gly Phe Ala Gly Phe Pro Val
   260 265 270
   Ser Gly Leu Trp Leu Arg Cys Thr Asn Glu Glu Leu Ile Glu Gln His
   275 280 285
   Ala Ala Lys Val Tyr Gly Lys Ala Ser Val Gly Ala Pro Pro Met Ser
   290 295 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
               340                 345                 350
   Leu Gly Val Ala Ala Gln Glu Phe Asp Leu Thr Lys Tyr Leu Val Thr
           355                 360                 365
   Glu Val Leu Lys Asp Gln Asp Lys Arg Met Asp Ala Leu Arg Glu Tyr
       370                 375                 380
   Met Pro Glu Ala Gln Asn Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
                   485                 490                 495
   Leu Glu Glu Ala
               500
<210> 11
   <211> 20
   <212> DNA
   <213> Artificial Sequence
<220>
   <223> Primer mqo-Start
<400> 11 cagagaactc gcggagataa 20
<210> 12
   <211> 20
   <212> DNA
   <213> Artificial Sequence
<220>
   <223> Primer mqo-Stop
<400> 12
   aacctcaaca gacatgtgcg 20
<210> 13
   <211> 20
   <212> DNA
   <213> Artificial Sequence
<220>
   <223> Primer mqo-A1
<400> 13
   ggtgaaactt ccgcgatact 20
<210> 14
   <211> 20
   <212> DNA
   <213> Artificial sequence
<220>
   <223> Primer mqo-E1
<400> 14
   gtgtcgccta aatcacactg 20
<210> 15
   <211> 99
   <212> DNA
   <213> Corynebacterium glutamicum
<220>
   <221> CDS
   <222> (1)..(99)
   <223> Leseraster
<220>
   <221> mutation
   <222> (49)..(51)
   <223> Position 49 bis 51 entspricht Position 331 bis 333 von SEQ ID NO:1, SEQ ID NO:5, SEQ ID NO:7 oder SEQ ID NO:9
<220>
   <221> misc_feature
   <222> (49)..(51)
   <223> n is a, c, g, or t
<400> 15
   cag gtt tcc cgt cag ttc tgg tct cac ctc gtt gaa gag gga gtg ctg        48
   Gln Val Ser Arg Gln Phe Trp Ser His Leu Val Glu Glu Gly Val Leu
   1               5                   10                  15
   nnn gat cct aag gaa ttc atc aac cct gtt cct cac gta tct ttc ggc        96
   Xaa Asp Pro Lys Glu Phe Ile Asn Pro Val Pro His Val Ser Phe Gly
               20                  25                  30
   cag                                                                    99
   Gln
<210> 16
   <211> 33
   <212> PRT
   <213> Corynebacterium glutamicum
<220>
   <221> misc_feature
   <222> (17)..(17)
   <223> The 'xaa' at location 17 stands for Lys, Asn, Arg, Thr, Ile, Met, Glu, Asp, Gly, Ala, val, Gln, His, Pro, Leu, Tyr, Trp, Cys, or Phe.
<400> 16
   Gln Val Ser Arg Gln Phe Trp Ser His Leu Val Glu Glu Gly Val Leu
   1               5                   10                  15
   Xaa Asp Pro Lys Glu Phe Ile Asn Pro Val Pro His Val Ser Phe Gly
               20                  25                  30
   Gln
<210> 17
   <211> 99
   <212> DNA
   <213> Corynebacterium glutamicum
<220>
   <221> CDS
   <222> (1)..(99)
   <223> Leseraster
<220>
   <221> mutation
   <222> (49)..(51)
   <223> Position 49 bis 51 entspricht Position 331 bis 333 von SEQ ID NO:5
<400> 17
   cag gtt tcc cgt cag ttc tgg tct cac ctc gtt gaa gag gga gtg ctg        48
   Gln Val Ser Arg Gln Phe Trp Ser His Leu Val Glu Glu Gly Val Leu
   1               5                   10                  15
   ttt gat cct aag gaa ttc atc aac cct gtt cct cac gta tct ttc ggc        96
   Phe Asp Pro Lys Glu Phe Ile Asn Pro Val Pro His Val Ser Phe Gly
               20                  25                  30
   cag                                                                    99
   Gln
<210> 18
   <211> 33
   <212> PRT
   <213> Corynebacterium glutamicum
<400> 18
   Gln Val Ser Arg Gln Phe Trp Ser His Leu Val Glu Glu Gly Val Leu
   1               5                   10                  15
   Phe Asp Pro Lys Glu Phe Ile Asn Pro Val Pro His Val Ser Phe Gly
               20                  25                  30
   Gln
<210> 19
   <211> 99
   <212> DNA
   <213> Corynebacterium glutamicum
<220>
   <221> CDS
   <222> (1)..(99)
   <223> Leseraster
<220>
   <221> mutation
   <222> (49)..(51)
   <223> Position 49 bis 51 entspricht Position 331 bis 333 von SEQ ID NO:7 oder SEQ ID NO:9
<400> 19
   cag gtt tcc cgt cag ttc tgg tct cac ctc gtt gaa gag gga gtg ctg        48
   Gln Val Ser Arg Gln Phe Trp Ser His Leu Val Glu Glu Gly Val Leu
   1               5                   10                  15
   gct gat cct aag gaa ttc atc aac cct gtt cct cac gta tct ttc ggc        96
   Ala Asp Pro Lys Glu Phe Ile Asn Pro Val Pro His Val Ser Phe Gly
               20                  25                  30
   cag                                                                    99
   Gln
<210> 20
   <211> 33
   <212> PRT
   <213> Corynebacterium glutamicum
<400> 20
   Gln Val Ser Arg Gln Phe Trp Ser His Leu Val Glu Glu Gly Val Leu
   1               5                   10                  15
   Ala Asp Pro Lys Glu Phe Ile Asn Pro Val Pro His Val Ser Phe Gly
               20                  25                  30
   Gln
<210> 21
   <211> 1263
   <212> DNA
   <213> Corynebacterium glutamicum
<220>
   <221> CDS
   <222> (1)..(1263)
   <223> lysc-wildtypgen
<400> 21
   gtg gcc ctg gtc gta cag aaa tat ggc ggt tcc tcg ctt gag agt gcg      48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct      96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat     144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt     192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc     240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg     288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc     336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc     384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc     432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg     480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt     528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag     576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc     624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat     672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg     720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc     768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att     816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat      864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa      912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc      960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc     1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct     1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg     1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt     1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca     1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat     1248
   Leu His Glu Gln Phe Gln Leu Gly Gly Glu Asp Glu Ala Val Val Tyr
                   405                 410                 415
   gca ggc acc gga cgc                                                 1263
   Ala Gly Thr Gly Arg
               420
<210> 22
   <211> 421
   <212> PRT
   <213> Corynebacterium glutamicum
<400> 22
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
   85 90 95
   Gly Ser Gln Ala Gly Val Leu Thr Thr Glu Arg His Gly Asn Ala Arg
   100 105 110
   Ile Val Asp Val Thr Pro Gly Arg Val Arg Glu Ala Leu Asp Glu Gly
   115 120 125
   Lys Ile Cys Ile Val Ala Gly Phe Gln Gly Val Asn Lys Glu Thr Arg
   130 135 140
   Asp Val Thr Thr Leu Gly Arg Gly Gly Ser Asp Thr Thr Ala Val Ala
   145 150 155 160
   Leu Ala Ala Ala Leu Asn Ala Asp Val Cys Glu Ile Tyr Ser Asp Val
   165 170 175
   Asp Gly Val Tyr Thr Ala Asp Pro Arg Ile Val Pro Asn Ala Gln Lys
   180 185 190
   Leu Glu Lys Leu Ser Phe Glu Glu Met Leu Glu Leu Ala Ala Val Gly
   195 200 205
   Ser Lys Ile Leu Val Leu Arg Ser Val Glu Tyr Ala Arg Ala Phe Asn
   210 215 220
   Val Pro Leu Arg Val Arg Ser Ser Tyr Ser Asn Asp Pro Gly Thr Leu
   225 230 235 240
   Ile Ala Gly Ser Met Glu Asp Ile Pro Val Glu Glu Ala Val Leu Thr
   245 250 255
   Gly Val Ala Thr Asp Lys Ser Glu Ala Lys Val Thr Val Leu Gly Ile
   260 265 270
   Ser Asp Lys Pro Gly Glu Ala Ala Lys Val Phe Arg Ala Leu Ala Asp
   275 280 285
   Ala Glu Ile Asn Ile Asp Met Val Leu Gln Asn Val Ser Ser Val Glu
   290 295 300
   Asp Gly Thr Thr Asp Ile Thr Phe Thr Cys Pro Arg Ser Asp Gly Arg
   305 310 315 320
   Arg Ala Met Glu Ile Leu Lys Lys Leu Gln Val Gln Gly Asn Trp Thr
   325 330 335
   Asn Val Leu Tyr Asp Asp Gln Val Gly Lys Val Ser Leu Val Gly Ala
   340 345 350
   Gly Met Lys Ser His Pro Gly Val Thr Ala Glu Phe Met Glu Ala Leu
   355 360 365
   Arg Asp Val Asn Val Asn Ile Glu Leu Ile Ser Thr Ser Glu Ile Arg
   370 375 380
   Ile Ser Val Leu Ile Arg Glu Asp Asp Leu Asp Ala Ala Ala Arg Ala
   385 390 395 400
   Leu His Glu Gln Phe Gln Leu Gly Gly Glu Asp Glu Ala Val Val Tyr
   405 410 415
   Ala Gly Thr Gly Arg
   420
<210> 23
   <211> 1503
   <212> DNA
   <213> Corynebacterium glutamicum
<220>
   <221> CDS
   <222> (1)..(1500)
   <223> mqo-Allel
<220>
   <221> mutation
   <222> (1168)..(1170)
<220>
   <221> misc_feature
   <222> (1168)..(1170)
   <223> n is a, c, g, or t
<400> 23
   atg tca gat tcc ccg aag aac gca ccg agg att acc gat gag gca gat      48
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg      96
   Val Val Leu Ile Gly Ala Gly Ile Met Ser Ser Thr Leu Gly Ala Met
               20                  25                  30
   ctg cgt cag ctg gag cca agc tgg act cag atc gtc ttc gag cgt ttg     144
   Leu Arg Gln Leu Glu Pro Ser Trp Thr Gln Ile Val Phe Glu Arg Leu
           35                  40                  45
   gat gga ccg gca caa gag tcg tcc tcc ccg tgg aac aat gca gga acc     192
   Asp Gly Pro Ala Gln Glu Ser Ser Ser Pro Trp Asn Asn Ala Gly Thr
       50                  55                  60
   ggc cac tct gct cta tgc gag ctg aac tac acc cca gag gtt aag ggc     240
   Gly His Ser Ala Leu Cys Glu Leu Asn Tyr Thr Pro Glu Val Lys Gly
   65                  70                  75                  80
   aag gtt gaa att gcc aag gct gta gga atc aac gag aag ttc cag gtt     288
   Lys Val Glu Ile Ala Lys Ala Val Gly Ile Asn Glu Lys Phe Gln Val
                   85                  90                  95
   tcc cgt cag ttc tgg tct cac ctc gtt gaa gag gga gtg ctg tct gat     336
   Ser Arg Gln Phe Trp Ser His Leu Val Glu Glu Gly Val Leu Ser Asp
               100                 105                 110
   cct aag gaa ttc atc aac cct gtt cct cac gta tct ttc ggc cag ggc     384
   Pro Lys Glu Phe Ile Asn Pro Val Pro His Val Ser Phe Gly Gln Gly
           115                 120                 125
   gca gat cag gtt gca tac atc aag gct cgc tac gaa gct ttg aag gat      432
   Ala Asp Gln Val Ala Tyr Ile Lys Ala Arg Tyr Glu Ala Leu Lys Asp
       130                 135                 140
   cac cca ctc ttc cag ggc atg acc tac gct gac gat gaa gct acc ttc      480
   His Pro Leu Phe Gln Gly Met Thr Tyr Ala Asp Asp Glu Ala Thr Phe
   145                 150                 155                 160
   acc gag aag ctg cct ttg atg gca aag ggc cgt gac ttc tct gat cca      528
   Thr Glu Lys Leu Pro Leu Met Ala Lys Gly Arg Asp Phe Ser Asp Pro
                   165                 170                 175
   gta gca atc tct tgg atc gat gaa ggc acc gac atc aac tac ggt gct      576
   Val Ala Ile Ser Trp Ile Asp Glu Gly Thr Asp Ile Asn Tyr Gly Ala
               180                 185                 190
   cag acc aag cag tac ctg gat gca gct gaa gtt gaa ggc act gaa atc      624
   Gln Thr Lys Gln Tyr Leu Asp Ala Ala Glu Val Glu Gly Thr Glu Ile
           195                 200                 205
   cgc tat ggc cac gaa gtc aag agc atc aag gct gat ggc gca aag tgg      672
   Arg Tyr Gly His Glu Val Lys Ser Ile Lys Ala Asp Gly Ala Lys Trp
       210                 215                 220
   atc gtg acc gtc aag aac gta cac act ggc gac acc aag acc atc aag      720
   Ile Val Thr Val Lys Asn Val His Thr Gly Asp Thr Lys Thr Ile Lys
   225                 230                 235                 240
   gca aac ttc gtg ttc gtc ggc gca ggc gga tac gca ctg gat ctg ctt      768
   Ala Asn Phe Val Phe Val Gly Ala Gly Gly Tyr Ala Leu Asp Leu Leu
                   245                 250                 255
   cgc agc gca ggc atc cca cag gtc aag ggc ttc gct gga ttc cca gta      816
   Arg Ser Ala Gly Ile Pro Gln Val Lys Gly Phe Ala Gly Phe Pro Val
               260                 265                 270
   tcc ggc ctg tgg ctt cgt tgc acc aac gag gaa ctg atc gag cag cac      864
   Ser Gly Leu Trp Leu Arg Cys Thr Asn Glu Glu Leu Ile Glu Gln His
           275                 280                 285
   gca gcc aag gta tat ggc aag gca tct gtt ggc gct cct cca atg tct      912
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc      960
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc     1008
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac     1056
   Tyr Leu Asp Leu Phe Lys Ser Ile Arg Pro Asp Asn Ile Pro Ser Tyr
               340                 345                 350
   ctt ggc gtt gct gct cag gaa ttt gat ctg acc aag tac ctt gtc act     1104
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac     1152
   Glu Val Leu Lys Asp Gln Asp Lys Arg Met Asp Ala Leu Arg Glu Tyr
       370                 375                 380
   atg cca gag gca caa nnn ggc gat tgg gag acc atc gtt gcc gga cag     1200
   Met Pro Glu Ala Gln Xaa Gly Asp Trp Glu Thr Ile Val Ala Gly Gln
   385                 390                 395                 400
   cgt gtt cag gtt att aag cct gca gga ttc cct aag ttc ggt tcc ctg     1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga     1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc     1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac     1392
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag     1440
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ctg aag     1488
   Pro Ala Leu Phe Glu Gln Gln Trp Ala Arg Thr Gln Lys Thr Leu Lys
                   485                 490                 495
   ctt gag gaa gcc taa                                                 1503
   Leu Glu Glu Ala
               500
<210> 24
   <211> 500
   <212> PRT
   <213> Corynebacterium glutamicum
<220>
   <221> misc_feature
   <222> (390)..(390)
   <223> The 'xaa' at location 390 stands for Lys, Arg, ser, Thr, Ile, Met, Glu, Asp, Gly, Ala, Val, Gln, His, Pro, Leu, Tyr, Trp, Cys, or Phe.
<400> 24
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
                   85                  90                  95
   Ser Arg Gln Phe Trp Ser His Leu Val Glu Glu Gly Val Leu Ser Asp
               100                 105                 110
   Pro Lys Glu Phe Ile Asn Pro Val Pro His Val Ser Phe Gly Gln Gly
           115                 120                 125
   Ala Asp Gln Val Ala Tyr Ile Lys Ala Arg Tyr Glu Ala Leu Lys Asp
       130                 135                 140
   His Pro Leu Phe Gln Gly Met Thr Tyr Ala Asp Asp Glu Ala Thr Phe
   145                 150                 155                 160
   Thr Glu Lys Leu Pro Leu Met Ala Lys Gly Arg Asp Phe Ser Asp Pro
                   165                 170                 175
   Val Ala Ile Ser Trp Ile Asp Glu Gly Thr Asp Ile Asn Tyr Gly Ala
               180                 185                 190
   Gln Thr Lys Gln Tyr Leu Asp Ala Ala Glu Val Glu Gly Thr Glu Ile
           195                 200                 205
   Arg Tyr Gly His Glu Val Lys Ser Ile Lys Ala Asp Gly Ala Lys Trp
       210                 215                 220
   Ile Val Thr Val Lys Asn Val His Thr Gly Asp Thr Lys Thr Ile Lys
   225                 230                 235                 240
   Ala Asn Phe Val Phe Val Gly Ala Gly Gly Tyr Ala Leu Asp Leu Leu
                   245                 250                 255
   Arg Ser Ala Gly Ile Pro Gln Val Lys Gly Phe Ala Gly Phe Pro Val
               260                 265                 270
   Ser Gly Leu Trp Leu Arg Cys Thr Asn Glu Glu Leu Ile Glu Gln His
           275                 280                 285
   Ala Ala Lys Val Tyr Gly Lys Ala Ser Val Gly Ala Pro Pro Met Ser
       290                 295                 300
   Val Pro His Leu Asp Thr Arg Val Ile Glu Gly Glu Lys Gly Leu Leu
   305                 310                 315                 320
   Phe Gly Pro Tyr Gly Gly Trp Thr Pro Lys Phe Leu Lys Glu Gly Ser
                   325                 330                 335
   Tyr Leu Asp Leu Phe Lys Ser Ile Arg Pro Asp Asn Ile Pro Ser Tyr
               340                 345                 350
   Leu Gly Val Ala Ala Gln Glu Phe Asp Leu Thr Lys Tyr Leu Val Thr
           355                 360                 365
   Glu Val Leu Lys Asp Gln Asp Lys Arg Met Asp Ala Leu Arg Glu Tyr
   370                 375                 380
   Met Pro Glu Ala Gln Xaa Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
                   485                 490                 495
   Leu Glu Glu Ala
               500
<210> 25
   <211> 1503
   <212> DNA
   <213> Corynebacterium glutamicum
<220>
   <221> CDS
   <222> (1)..(1500)
   <223> mqo-Allel
<220>
   <221> mutation
   <222> (1168)..(1170)
<400> 25
   atg tca gat tcc ccg aag aac gca ccg agg att acc gat gag gca gat      48
   Met Ser Asp Ser Pro Lys Asn Ala Pro Arg Ile Thr Asp Glu Ala Asp
   1               5                   10                  15
   gta gtt ctc att ggt gcc ggt atc atg agc tcc acg ctg ggt gca atg      96
   Val Val Leu Ile Gly Ala Gly Ile Met Ser Ser Thr Leu Gly Ala Met
               20                  25                  30
   ctg cgt cag ctg gag cca agc tgg act cag atc gtc ttc gag cgt ttg     144
   Leu Arg Gln Leu Glu Pro Ser Trp Thr Gln Ile Val Phe Glu Arg Leu
           35                  40                  45
   gat gga ccg gca caa gag tcg tcc tcc ccg tgg aac aat gca gga acc     192
   Asp Gly Pro Ala Gln Glu Ser Ser Ser Pro Trp Asn Asn Ala Gly Thr
       50                  55                  60
   ggc cac tct gct cta tgc gag ctg aac tac acc cca gag gtt aag ggc     240
   Gly His Ser Ala Leu Cys Glu Leu Asn Tyr Thr Pro Glu Val Lys Gly
   65                  70                  75                  80
   aag gtt gaa att gcc aag gct gta gga atc aac gag aag ttc cag gtt      288
   Lys Val Glu Ile Ala Lys Ala Val Gly Ile Asn Glu Lys Phe Gln Val
                   85                  90                  95
   tcc cgt cag ttc tgg tct cac ctc gtt gaa gag gga gtg ctg tct gat      336
   Ser Arg Gln Phe Trp Ser His Leu Val Glu Glu Gly Val Leu Ser Asp
               100                 105                 110
   cct aag gaa ttc atc aac cct gtt cct cac gta tct ttc ggc cag ggc      384
   Pro Lys Glu Phe Ile Asn Pro Val Pro His Val Ser Phe Gly Gln Gly
           115                 120                 125
   gca gat cag gtt gca tac atc aag gct cgc tac gaa gct ttg aag gat      432
   Ala Asp Gln Val Ala Tyr Ile Lys Ala Arg Tyr Glu Ala Leu Lys Asp
       130                 135                 140
   cac ccc ctc ttc cag ggc atg acc tac gct gac gat gaa gct acc ttc      480
   His Pro Leu Phe Gln Gly Met Thr Tyr Ala Asp Asp Glu Ala Thr Phe
   145                 150                 155                 160
   acc gag aag ctg cct ttg atg gca aag ggc cgt gac ttc tct gat cca      528
   Thr Glu Lys Leu Pro Leu Met Ala Lys Gly Arg Asp Phe Ser Asp Pro
                   165                 170                 175
   gta gca atc tct tgg atc gat gaa ggc acc gac atc aac tac ggt gct      576
   Val Ala Ile Ser Trp Ile Asp Glu Gly Thr Asp Ile Asn Tyr Gly Ala
               180                 185                 190
   cag acc aag cag tac ctg gat gca gct gaa gtt gaa ggc act gaa atc      624
   Gln Thr Lys Gln Tyr Leu Asp Ala Ala Glu Val Glu Gly Thr Glu Ile
           195                 200                 205
   cgc tat ggc cac gaa gtc aag agc atc aag gct gat ggc gca aag tgg      672
   Arg Tyr Gly His Glu Val Lys Ser Ile Lys Ala Asp Gly Ala Lys Trp
       210                 215                 220
   atc gtg acc gtc aag aac gta cac act ggc gac acc aag acc atc aag      720
   Ile Val Thr Val Lys Asn Val His Thr Gly Asp Thr Lys Thr Ile Lys
   225                 230                 235                 240
   gca aac ttc gtg ttc gtc ggc gca ggc gga tac gca ctg gat ctg ctt      768
   Ala Asn Phe Val Phe Val Gly Ala Gly Gly Tyr Ala Leu Asp Leu Leu
                   245                 250                 255
   cgc agc gca ggc atc cca cag gtc aag ggc ttc gct gga ttc cca gta      816
   Arg Ser Ala Gly Ile Pro Gln Val Lys Gly Phe Ala Gly Phe Pro Val
               260                 265                 270
   tcc ggc ctg tgg ctt cgt tgc acc aac gag gaa ctg atc gag cag cac      864
   Ser Gly Leu Trp Leu Arg Cys Thr Asn Glu Glu Leu Ile Glu Gln His
           275                 280                 285
   gca gcc aag gta tat ggc aag gca tct gtt ggc gct cct cca atg tct      912
   Ala Ala Lys Val Tyr Gly Lys Ala Ser Val Gly Ala Pro Pro Met Ser
       290                 295                 300
   gtt cct cac ctt gac acc cgc gtt atc gag ggt gaa aag ggt ctg ctc      960
   Val Pro His Leu Asp Thr Arg Val Ile Glu Gly Glu Lys Gly Leu Leu
   305                 310                 315                 320
   ttt gga cct tac ggt ggc tgg acc cct aag ttc ttg aag gaa ggc tcc     1008
   Phe Gly Pro Tyr Gly Gly Trp Thr Pro Lys Phe Leu Lys Glu Gly Ser
                   325                 330                 335
   tac ctg gac ctg ttc aag tcc atc cgc cca gac aac att cct tcc tac     1056
   Tyr Leu Asp Leu Phe Lys Ser Ile Arg Pro Asp Asn Ile Pro Ser Tyr
                   340                 345                 350
   ctt ggc gtt gct gct cag gaa ttt gat ctg acc aag tac ctt gtc act      1104
   Leu Gly Val Ala Ala Gln Glu Phe Asp Leu Thr Lys Tyr Leu Val Thr
           355                 360                 365
   gaa gtt ctc aag gac cag gac aag cgt atg gat gct ctt cgc gag tac      1152
   Glu Val Leu Lys Asp Gln Asp Lys Arg Met Asp Ala Leu Arg Glu Tyr
       370                 375                 380
   atg cca gag gca caa tac ggc gat tgg gag acc atc gtt gcc gga cag      1200
   Met Pro Glu Ala Gln Tyr Gly Asp Trp Glu Thr Ile Val Ala Gly Gln
   385                 390                 395                 400
   cgt gtt cag gtt att aag cct gca gga ttc cct aag ttc ggt tcc ctg      1248
   Arg Val Gln Val Ile Lys Pro Ala Gly Phe Pro Lys Phe Gly Ser Leu
                   405                 410                 415
   gaa ttc ggc acc acc ttg atc aac aac tcc gaa ggc acc atc gcc gga      1296
   Glu Phe Gly Thr Thr Leu Ile Asn Asn Ser Glu Gly Thr Ile Ala Gly
               420                 425                 430
   ttg ctc ggt gct tcc cct gga gca tcc atc gca cct tcc gca atg atc      1344
   Leu Leu Gly Ala Ser Pro Gly Ala Ser Ile Ala Pro Ser Ala Met Ile
           435                 440                 445
   gag ctg ctt gag cgt tgc ttc ggt gac cgc atg atc gag tgg ggc gac      1392
   Glu Leu Leu Glu Arg Cys Phe Gly Asp Arg Met Ile Glu Trp Gly Asp
       450                 455                 460
   aag ctg aag gac atg atc cct tcc tac ggc aag aag ctt gct tcc gag      1440
   Lys Leu Lys Asp Met Ile Pro Ser Tyr Gly Lys Lys Leu Ala Ser Glu
   465                 470                 475                 480
   cca gca ctg ttt gag cag cag tgg gca cgc acc cag aag acc ctg aag      1488
   Pro Ala Leu Phe Glu Gln Gln Trp Ala Arg Thr Gln Lys Thr Leu Lys
                   485                 490                 495
   ctt gag gaa gcc taa                                                  1503
   Leu Glu Glu Ala
               500
<210> 26
   <211> 500
   <212> PRT
   <213> Corynebacterium glutamicum
<400> 26
   Met Ser Asp Ser Pro Lys Asn Ala Pro Arg Ile Thr Asp Glu Ala Asp
   1               5                   10                  15
   Val Val Leu Ile Gly Ala Gly Ile Met Ser Ser Thr Leu Gly Ala Met
               20                  25                  30
   Leu Arg Gln Leu Glu Pro Ser Trp Thr Gln Ile Val Phe Glu Arg Leu
           35                  40                  45
   Asp Gly Pro Ala Gln Glu Ser Ser Ser Pro Trp Asn Asn Ala Gly Thr
       50                  55                  60
   Gly His Ser Ala Leu Cys Glu Leu Asn Tyr Thr Pro Glu Val Lys Gly
   65                  70                  75                  80
   Lys Val Glu Ile Ala Lys Ala Val Gly Ile Asn Glu Lys Phe Gln Val
   85 90 95
   Ser Arg Gln Phe Trp Ser His Leu Val Glu Glu Gly Val Leu Ser Asp
   100 105 110
   Pro Lys Glu Phe Ile Asn Pro Val Pro His Val Ser Phe Gly Gln Gly
   115 120 125
   Ala Asp Gln Val Ala Tyr Ile Lys Ala Arg Tyr Glu Ala Leu Lys Asp
   130 135 140
   His Pro Leu Phe Gln Gly Met Thr Tyr Ala Asp Asp Glu Ala Thr Phe
   145 150 155 160
   Thr Glu Lys Leu Pro Leu Met Ala Lys Gly Arg Asp Phe Ser Asp Pro
   165 170 175
   Val Ala Ile Ser Trp Ile Asp Glu Gly Thr Asp Ile Asn Tyr Gly Ala
   180 185 190
   Gln Thr Lys Gln Tyr Leu Asp Ala Ala Glu Val Glu Gly Thr Glu Ile
   195 200 205
   Arg Tyr Gly His Glu Val Lys Ser Ile Lys Ala Asp Gly Ala Lys Trp
   210 215 220
   Ile Val Thr Val Lys Asn Val His Thr Gly Asp Thr Lys Thr Ile Lys
   225 230 235 240
   Ala Asn Phe Val Phe Val Gly Ala Gly Gly Tyr Ala Leu Asp Leu Leu
   245 250 255
   Arg Ser Ala Gly Ile Pro Gln Val Lys Gly Phe Ala Gly Phe Pro Val
   260 265 270
   Ser Gly Leu Trp Leu Arg Cys Thr Asn Glu Glu Leu Ile Glu Gln His
   275 280 285
   Ala Ala Lys Val Tyr Gly Lys Ala Ser Val Gly Ala Pro Pro Met Ser
   290 295 300
   Val Pro His Leu Asp Thr Arg Val Ile Glu Gly Glu Lys Gly Leu Leu
   305 310 315 320
   Phe Gly Pro Tyr Gly Gly Trp Thr Pro Lys Phe Leu Lys Glu Gly Ser
   325 330 335
   Tyr Leu Asp Leu Phe Lys Ser Ile Arg Pro Asp Asn Ile Pro Ser Tyr
   340 345 350
   Leu Gly Val Ala Ala Gln Glu Phe Asp Leu Thr Lys Tyr Leu Val Thr
           355                 360                 365
   Glu Val Leu Lys Asp Gln Asp Lys Arg Met Asp Ala Leu Arg Glu Tyr
       370                 375                 380
   Met Pro Glu Ala Gln Tyr Gly Asp Trp Glu Thr Ile Val Ala Gly Gln
   385                 390                 395                 400
   Arg Val Gln Val Ile Lys Pro Ala Gly Phe Pro Lys Phe Gly Ser Leu
                   405                 410                 415
   Glu Phe Gly Thr Thr Leu Ile Asn Asn Ser Glu Gly Thr Ile Ala Gly
               420                 425                 430
   Leu Leu Gly Ala Ser Pro Gly Ala Ser Ile Ala Pro Ser Ala Met Ile
           435                 440                 445
   Glu Leu Leu Glu Arg Cys Phe Gly Asp Arg Met Ile Glu Trp Gly Asp
       450                 455                 460
   Lys Leu Lys Asp Met Ile Pro Ser Tyr Gly Lys Lys Leu Ala Ser Glu
   465                 470                 475                 480
   Pro Ala Leu Phe Glu Gln Gln Trp Ala Arg Thr Gln Lys Thr Leu Lys
                   485                 490                 495
   Leu Glu Glu Ala
<210> 27
   <211> 30
   <212> DNA
   <213> Artificial Sequence
<220>
   <223> Primer mqo-int1-bam
<400> 27
   ctagggatcc ccgaagaacg caccgaggat 30
<210> 28
   <211> 30
   <212> DNA
   <213> Artificial Sequence
<220>
   <223> Primer mqo-int2-bam
<400> 28
   ctagggatcc ggcggatgga cttgaacagg 30

## Beschreibung

Gegenstand der Erfindung sind Mutanten und Allele des mqo-Gens coryneformer Bakterien kodierend für Varianten der Malat-Chinon-Oxidoreduktase (EC: 1.1.99.16) und Verfahren zur Herstellung von Aminosäuren, insbesondere L-Lysin, Tryptophan und L-Prolin, unter Verwendung von Bakterien, die diese Allele enthalten.

### Stand der Technik

Aminosäuren finden in der Humanmedizin, in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen von Corynebacterium eingesetzt, indem man einzelne Aminosäure-Biosynthesegene amplifiziert und die Auswirkung auf die AminosäureProduktion untersucht. Eine zusammenfassende Darstellung über verschiedenste Aspekte der Genetik, des Stoffwechsels und der Biotechnologie von Corynebacterium glutamicum findet sich bei Pühler (chief ed.) im Journal of Biotechnology 104 (1-3), 1-338, 2003.

Die Nukleotidsequenz des für die Malat-Chinon-Oxidoreduktase von Corynebacterium glutamicum kodierenden Gens wurde von Molenaar et al.(European Journal of Biochemistry 254 : 395-403 (1998)) bestimmt und ist in der Datenbank des National Center for Biotechnology Information (NCBI) der National Library of Medicin (Bethesda, MD, USA) unter der Zugangsnummer (accession number) AJ224946 allgemein verfügbar.

Sie kann weiterhin der Patentanmeldung WO 01/00844 als Sequenz Nr. 569 und als Sequenz Nr. 571 sowie der Patentanmeldung EP-A-1108790 als Sequenz Nr. 3478, Sequenz Nr. 7065 und als Sequenz Nr. 7066 entnommen werden.

In der EP1038969 ist eine Verbesserung der fermentativen Produktion von L-Aminosäuren durch coryneforme Bakterien durch die Verstärkung des Gens mqo beschrieben.

In der WO02086137 wird dagegen die förderliche Wirkung der Abschwächung des Gens mqo auf die L-Aminosäureproduktion durch coryneforme Bakterien beschrieben. Eine in der Anmeldung beschriebene Mutation des mqo-Gens, bezeichnet mit "Allel 672" , trägt das Nukleotid Adenin anstelle des Nukleotids Guanin an Position 672 der DNA-Sequenz des mqo-Gens, was zur Entstehung eines Stop-Kodons an Position 224 der Aminosäure-Sequenz der Malat-Chinon-Oxidoreduktase von Corynebacterium glutamicum führt. Es wird ebenfalls das "Allel 1230" beschrieben, welches zusätzlich zu der Mutation in Allel 672 noch eine Cytosin zu Thymin Transition an Position 1230 der Nukleotidsequenz des mqo-Gens enthält. Weiterhin ist in der Anmeldung das Ausschalten des mqo-Gens durch Gen-Unterbrechung mittels Integrationsmutagenese beschrieben, welche zu einer Steigerung der Produktion von L-Lysin des entsprechenden Stammes führt.

Die mikrobielle Biosynthese von L-Aminosäuren in coryneformen Bakterien ist ein komplexes System und vielschichtig mit diversen anderen Stoffwechselwegen in der Zelle vernetzt. Daher kann keine Vorhersage gemacht werden, ob ein völliges Ausschalten oder ein Herabsenken der katalytischen Aktivität der Malat-Chinon-Oxidoreduktase auf verschiedene Stufen die Produktion von L-Aminosäuren verbessert. Es ist daher wünschenswert, auch Varianten der Malat-Chinon-Oxidoreduktase, die sich im Grad ihrer Aktivität unterscheiden, zur Verfügung zu haben.

Der besseren Übersichtlichkeit halber ist die Nukleotidsequenz des für die Malat-Chinon-Oxidoreduktase aus Corynebacterium glutamicum kodierenden mqo-Gens ("Wildtyp-Gen") gemäß den Angaben der NCBI-Datenbank in SEQ ID NO:1 und die sich daraus ergebende Aminosäuresequenz der kodierten Malat-Chinon-Oxidoreduktase in SEQ ID NO:2 und 4 dargestellt. In SEQ ID NO:3 sind stromaufwärts (upstream) und stromabwärts (downstream) gelegene Nukleotidsequenzen mit angegeben.

### Aufgabe der Erfindung

Die Erfinder haben sich zur Aufgabe gestellt, neue Maßnahmen zur verbesserten Herstellung von Aminosäuren, insbesondere L-Lysin, L-Tryptophan und L-Prolin, bereitzustellen.

### Beschreibung der Erfindung

Gegenstand der Erfindung sind erzeugte beziehungsweise isolierte Mutanten coryneformer Bakterien, die bevorzugt Aminosäuren ausscheiden, und welche ein Gen bzw. Allel enthalten, ausgewählt aus einem der folgenden a) bis c):
a) ein Gen, das für ein Polypeptid mit Malat-Chinon-Oxidoreduktase Aktivität kodiert, dadurch gekennzeichnet, dass das Polypeptid die Aminosäuresequenz SEQ ID NO: 2 umfasst, bei der an der Position 111 der Aminosäuresequenz von SEQ ID NO: 2 L-Phenylalanin enthalten ist;
b) ein Gen, das für ein Polypeptid mit Malat-Chinon-Oxidoreduktase Aktivität kodiert, wobei das kodierte Polypeptid eine Aminosäuresequenz umfasst, die mindestens 98 % oder mindestens 99 % identisch ist mit der Aminosäuresequenz von SEQ ID NO: 6, wobei in der Aminosäuresequenz an der Position, die der Position 111 der Aminosäuresequenz SEQ ID NO: 6 entspricht, L-Phenylalanin enthalten ist;
c) ein Gen, das für ein Protein mit Malat-Chinon-Oxidoreduktase Aktivität kodiert, wobei das kodierte Portein eine Aminosäuresequenz gemäß SEQ ID NO: 6 einschließlich eines bis fünf, bevorzugt höchstens zwei, höchstens drei oder höchstens vier, konservativer Aminosäureaustausch(e) umfasst, wobei in der Aminosäuresequenz an der Position, die der Position 111 der Aminosäuresequenz SEQ ID NO: 6 entspricht, L-Phenylalanin enthalten ist;
wobei die Aminosäuresekretion dieser Mutanten im Vergleich zum nicht-mutagenisierten Ausgangsstamm mit der Aminosäuresequenz gemäß SEQ ID NO: 2 um mindestens 0,5 % erhöht ist.

Bei den coryneformen Bakterien wird die Gattung Corynebacterium bevorzugt. Besonders bevorzugt sind Aminosäure-ausscheidende Stämme, die auf folgenden Arten beruhen:
Corynebacterium efficiens, wie zum Beispiel der Stamm DSM44549,
Corynebacterium glutamicum, wie zum Beispiel der Stamm ATCC13032,
Corynebacterium thermoaminogenes wie zum Beispiel der Stamm FERM BP-1539, und
Corynebacterium ammoniagenes, wie zum Beispiel der Stamm ATCC6871,
wobei die Art Corynbacterium glutamicum ganz besonders bevorzugt wird.

Einige Vertreter der Art Corynebacterium glutamicum sind im Stand der Technik auch unter anderen Artbezeicnungen bekannt. Hierzu gehören beispielsweise:
Corynebacterium acetoacidophilum ATCC13870
Corynebacterium lilium DSM20137
Corynebacterium melassecola ATCC17965
Brevibacterium flavum ATCC14067
Brevibacterium lactofermentum ATCC13869 und
Brevibacterium divaricatum ATCC14020

Bekannte Vertreter Aminosäure ausscheidender Stämme coryneformer Bakterien sind beispielsweise
die L-Lysin produzierenden Stämme
Corynebacterium glutamicum DM58-1/pDM6 (= DSM4697) beschrieben in EP 0 358 940
Corynebacterium glutamicum MH20 (= DSM5714) beschrieben in EP 0 435 132
Corynebacterium glutamicum AHP-3 (= FermBP-7382) beschrieben in EP 1 108 790
Corynebacterium thermoaminogenes AJ12521 (= FERM BP-3304) beschrieben in US 5,250,423 oder die L-Tryptophan produzierenden Stämme
Corynebacterium glutamicum K76 (=FermBP-1847) beschrieben in US 5,563,052
Corynebacterium glutamicum BPS13 (=FermBP-1777) beschrieben in US 5,605,818
Corynebacterium glutamicum FermBP-3055 beschrieben in US 5,235,940

Angaben zur taxonomischen Einordnung von Stämmen dieser Gruppe von Bakterien findet man unter anderem bei Seiler (Journal of General Microbiology 129, 1433-1477 (1983), Kämpfer und Kroppenstedt (Canadian Journal of Microbiology 42, 989-1005 (1996)), Liebl et al (International Journal of Systematic Bacteriology 41, 255-260 (1991) und in der US-A-5,250,434.

Stämme mit der Bezeichnung "ATCC" können von der American Type Culture Collection (Manassas, VA, USA) bezogen werden. Stämme mit der Bezeichnung "DSM" können von der Deutschen Sammlung von Mikroorganismen und Zellkulturen (DSMZ, Braunschweig, Deutschland) bezogen werden. Stämme mit der Bezeichnung "FERM" können vom National Institute of Advanced Industrial Science and Technology (AIST Tsukuba Central 6, 1-1-1 Higashi, Tsukuba Ibaraki, Japan) bezogen werden. Die genannten Stämme von Corynebacterium thermoaminogenes (FERM BP-1539, FERM BP-1540, FERM BP-1541 und FERM BP-1542) sind in der US-A 5,250,434 beschrieben.

Unter proteinogenen Aminosäuren versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin.

Die erfindungsgemäßen Mutanten scheiden bevorzugt die genannten proteinogen Aminosäuren, insbesondere L-Lysin aus. Der Begriff Aminosäuren umfasst auch deren Salze wie beispielsweise das Lysin-Monohydrochlorid oder Lysin-Sulfat im Falle der Aminosäure L-Lysin.

Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Aparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Bei der Arbeit an der vorliegenden Erfindung wurde durch Vergleich der Aminosäuresequenz mit dem Clustal-Programm (Thompson et al., Nucleic Acids Research 22, 4637-4680 (1994)) festgestellt, dass die Aminosäuresequenzen der Malat-Chinon-Oxidoreduktase verschiedener Bakterien wie bespielsweise Escherichia coli, Corynebacterium glutamicum, Corynebacterium efficiens, Corynebacterium diphteriae, Mycobacterium tuberculosis, Bacillus cereus und Bacillus halodurans ein Sequenzmotiv bestehend aus der Abfolge Trp-Asn-Asn-Ala-Gly-Thr-Gly-His-Sal-Ala-Leu und auch ein Sequenzmotiv bestehend aus der Abfolge Bra-Leu-Gly-Ali-Ser-Pro-Gly-Ala-Ser enthalten. Die Bezeichnung "Bra" steht für die Aminosäuren Ile oder Leu, die Bezeichnung "Sal" für die Aminosäuren Ser oder Ala und die Bezeichnung "Ali" für die Aminosäuren Ala oder Gly.

Dementsprechend sind solche Mutanten coryneformer Bakterien bevorzugt, die ein mqo-Allel enthalten, das für ein Polypeptid mit Malat-Chinon-Oxidoreduktase Enzymaktivität kodiert, welches mindestens eine Aminosäuresequenz ausgewählt aus der Gruppe Trp-Asn-Asn-Ala-Gly-Thr-Gly-His-Sal-Ala-Leu und Bra-Leu-Gly-Ali-Ser-Pro-Gly-Ala-Ser umfasst und an Position 111 beziehungsweise der entsprechenden oder vergleichbaren Position der Aminosäuresequenz L-Phenylalanin enthält. Gegebenenfalls enthält die Aminosäuresequenz darüberhinaus einen Aminosäureaustausch L-Alanin gegen L-Serin an der Position 201 gemäß SEQ ID NO:2.

Das Aminosäuresequenzmotiv Trp-Asn-Asn-Ala-Gly-Thr-Gly-His-Sal-Ala-Leu ist beispielsweise in der SEQ ID NO:6, 8 oder 10 von Position 59 bis 69 enthalten. Das Aminosäuresequenzmotiv Bra-Leu-Gly-Ali-Ser-Pro-Gly-Ala-Ser ist beispielsweise in der SEQ ID NO:6, 8 oder 10 von Position 433 bis 441 enthalten.

Gegenstand der Erfindung sind schließlich Mutanten coryneformer Bakterien, die ein mqo-Allel enthalten, das für ein Polypeptid mit Malat-Chinon-Oxidoreduktase Enzymaktivität kodiert, welches die Aminosäuresequenz von SEQ ID NO:6 umfasst.

Es ist bekannt, dass durch wirtseigene Enzyme, sogenannte Aminopeptidasen, das endständige Methionin bei der Proteinsynthese entfernt wird.

Unter dem Begriff "einer zu Position 111 der Aminosäuresequenz entsprechenden Position" oder "einer zu Position 111 der Aminosäuresequenz vergleichbaren Position" versteht man die Tatsache, dass durch Insertion oder Deletion eines für eine Aminosäure kodierenden Kodons im N-terminalen Bereich (bezogen auf die Position 111 von SEQ ID NO:6) des kodierten Polypeptids die Positionsangabe und Längenangabe im Falle einer Insertion formal um eine Einheit erhöht oder im Falle einer Deletion um eine Einheit verringert wird. Beispielsweise rückt durch Deletion des für die Aminosäure L-Serin kodierenden TCA-Kodons an Position 2 von SEQ ID NO:5 das L-Phenylalanin von Position 111 an Position 110. Die Längenangabe wäre dann: 499 Aminosäuren. In gleicher Weise wird durch Insertion oder Deletion eines für eine Aminosäure kodierenden Kodons im C-terminalen Bereich (bezogen auf die Position 111) des kodierten Polypeptids die Längenangabe im Falle einer Insertion formal um eine Einheit erhöht oder im Falle einer Deletion um eine Einheit verringert. Derartige vergleichbare Positionen lassen sich durch Vergleich der Aminosäuresequenzen in Form eines "alignments" beispielsweise mit Hilfe des Clustal-Programmes leicht identifizieren. Durch derartige Insertionen und Deletionen wird die enzymatische Aktivität im wesentlichen nicht berührt. "Im wesentlichen nicht berührt" bedeutet, dass sich die enzymatische Aktivität der genannten Varianten um maximal 10%, maximal 7,5%, maximal 5%, maximal 2,5% oder maximal 1% von der Aktivität des Polypeptids mit der Aminosäuresequenz von SEQ ID NO:6 unterscheidet.

Zur Herstellung der erfindungsgemäßen Mutanten können klassische in-vivo Mutageneseverfahren mit Zellpopulationen coryneformer Bakterien unter Verwendung mutagener Stoffe wie beispielsweise N-Methyl-N'-Nitro-N-Nitrosoguanidin (MNNG) oder ultraviolettes Licht verwendet werden. Mutagenesemethoden sind beispielsweise im Manual of Methods for General Bacteriology (Gerhard et al. (Eds.), American Society for Microbiology, Washington, DC, USA, 1981) oder bei Tosaka et al. (Agricultural and Biological Chemistry 42(4), 745-752 (1978)) oder bei Konicek et al (Folia Microbiologica 33, 337-343 (1988)) beschrieben. Typische Mutagenesen unter Verwendung von MNNG umfassen Konzentrationen von 50 bis 500 mg/l oder auch höhere Konzentrationen bis zu maximal 1 g/l, eine Inkubationszeit von 1 bis 30 Minuten bei einem pH von 5,5 bis 7,5. Unter diesen Bedingungen wird die Zahl der lebensfähigen Zellen um einen Anteil von ca. 50% bis 90% oder ca. 50% bis 99% oder ca. 50% bis 99,9% oder mehr reduziert.

Aus der mutagenisierten Zellpopulation werden Mutanten beziehungsweise Zellen entnommen und vermehrt. In einem weiteren Schritt werden die Zellen beispielsweise unter Zuhilfenahme eines Ribolysers (Hybaid, Heidelberg, Deutschland) aufgeschlossen und die intrazelluläre Malat-Chinon-Oxidoreduktase Aktivität beispielsweise nach der Methode von Molenaar et al.(European Journal of Biochemistry 254 : 395-403 (1998)) bestimmt. Auf diese Weise werden Mutanten identifiziert, deren intrazelluläre Malat-Chinon-Oxidoreduktase Aktivität um mindestens 30%, bevorzugt mindestens 35% und ganz besonders bevorzugt um mindestens 40% im Vergleich zum nicht mutagenisierten Ausgangsstamm verringert ist. Anschließend wird deren Fähigkeit untersucht, in einer Satzkultur (batch) unter Verwendung eines geeigneten Nährmediums Aminosäuren, bevorzugt L-Lysin, L-Tryptophan oder L-Prolin, auszuscheiden. Geeignete Nährmedien und Testbedingungen sind unter anderem in der US 6,221,636, in der US 5,840,551, in der US 5,770,409, in der US 5,605,818, in der US 5,275,940 und in der US 4,224,409 beschrieben. Bei Verwendungen von geeigneten Roboteranlagen, wie beispielsweise bei Zimmermann et al. (VDI Berichte Nr. 1841, VDI-Verlag, Düsseldorf, Deutschland 2004, 439-443) oder Zimmermann (Chemie Ingenenieur Technik 77 (4), 426-428 (2005))beschrieben, können zahlreiche Mutanten in kurzer Zeit untersucht werden. Auf diese Weise werden Mutanten identifiziert, die eine verringerte Malat-Chinon-Oxidoreduktase Aktivität besitzen und die im Vergleich zum Elternstamm beziehungsweise nicht mutagenisierten Ausgangsstamm vermehrt Aminosäuren in das Nährmedium ausscheiden. Dazu gehören erfindungsgemäße Mutanten, deren Aminosäuresekretion um mindestens 0,5% erhöht ist.

Anschließend wird aus den Mutanten DNA bereitgestellt beziehungsweise isoliert und mit Hilfe der Polymerase-Kettenreaktion unter Verwendung von Primerpaaren, die die Amplifizierung des mqo-Gens beziehungsweise des erfindungsgemäßen mqo-Allels oder der erfindungsgemäßen Mutation an Position 111 der Aminosäuresequenz erlauben, das entsprechende Polynukleotid synthetisiert. Vorzugsweise wird die DNA aus solchen Mutanten isoliert, die in erhöhter Weise Aminosäuren ausscheiden.

Hierzu können beliebige Primerpaare aus der stromaufwärts und stromabwärts der erfindungsgemäßen Mutation gelegenen Nukleotidsequenz und der dazu komplementären Nukleotidsequenz ausgewählt werden. Ein Primer eines Primerpaares umfasst hierbei bevorzugt mindestens 15, mindestens 18, mindestens 20, mindestens 21 oder mindestens 24 aufeinanderfolgende Nukleotide ausgewählt aus der Nukleotidsequenz zwischen Position 1 und 679 von SEQ ID NO:3. Der dazugehörige zweite Primer eines Primerpaares umfasst mindestens 15, mindestens 18, mindestens 20, mindestens 21 oder mindestens 24 aufeinanderfolgende Nukleotide ausgewählt aus der komplementären Nukleotidsequenz von Position 2002 und 953 von SEQ ID NO:3. Ist die Amplifikation der Kodierregion gewünscht, so wird das Primerpaar vorzugsweise aus der Nukleotidsequenz zwischen Position 1 und 349 von SEQ ID NO:3 und aus der komplementären Nukleotidsequenz zwischen Position 2002 und 1850 von SEQ ID NO:3 ausgewählt. Ist die Amplifikation eines Teils der Kodierregion, wie beispielsweise in SEQ ID NO:15, 17 und 19 dargestellt, erwünscht, so wird das Primerpaar vorzugsweise aus der Nukleotidsequenz zwischen Position 351 und 679 von SEQ ID NO:3 und aus der komplementären Nukleotidsequenz zwischen Position 1848 und 953 von SEQ ID NO:3 ausgewählt.

Geeignete Primerpaare sind beispielsweise das unter SEQ ID NO:11 und SEQ ID NO:12 wiedergegebene Primerpaar mqo-Start und mqo-Stop oder das unter SEQ ID NO:13 und SEQ ID NO:14 wiedergegebene Primerpaar mqo-A1 und mqo-E1.

Der Primer kann überdies mit Erkennungsstellen für Restriktionsenzyme, mit einer Biotin-Gruppe oder weiteren Accessoirs, wie sie im Stand der Technik beschrieben sind, ausgerüstet sein. Die Gesamtlänge des Primers beträgt im Allgemeinen maximal 30, 40, 50 oder 60 Nukleotide.

Für die Herstellung von Polynukleotiden durch Amplifikation ausgewählter Sequenzen wie dem erfindungsgemäßen mqo-Allel aus vorgelegter, beispielsweise chromosomaler DNA durch Amplifikation mittels PCR werden im Allgemeinen thermostabile DNA-Polymerasen eingesetzt. Beispiele derartiger DNA-Polymerasen sind die Taq-Polymerase aus Thermus aquaticus, die unter anderem von der Firma Qiagen (Hilden, Deutschland) vertrieben wird, die Vent-Polymerase aus Thermococcus litoralis, die unter anderem von der Firma New England Biolabs (Frankfurt, Deutschland) vertrieben wird oder die Pfu-Polymerase aus Pyrococcus furiosus, die unter anderem von der Firma Stratagene (La Jolla, USA) vertrieben wird. Bevorzugt werden Polymerasen mit "proof-reading"-Aktivität. "proof-reading"-Aktivität bedeutet, dass diese Polymerasen in der Lage sind, fehlerhaft eingebaute Nukleotide zu erkennen und den Fehler durch erneute Polymerisation zu beheben (Lottspeich und Zorbas, Bioanalytik, Spektrum Akademischer Verlag, Heidelberg, Deutschland (1998)). Beispiele für Polymerasen mit "proof-reading"-Aktivität sind die Vent-Polymerase und die Pfu-Polymerase.

Die Bedingungen im Reaktionsansatz werden nach Angaben des Herstellers eingestellt. Die Polymerasen werden vom Hersteller im Allgemeinen zusammen mit dem gebräuchlichen Puffer geliefert, welcher üblicherweise Konzentrationen von 10 - 100 mM Tris/HCl und 6 - 55 mM KCl bei pH 7,5 - 9,3 aufweist. Magnesiumchlorid wird in einer Konzentration von 0,5 - 10 mM zugesetzt, falls es nicht in dem vom Hersteller gelieferten Puffer enthalten ist. Dem Reaktionsansatz werden weiterhin Desoxynucleosidtriphosphate in einer Konzentration von 0,1 - 16,6 mM zugesetzt. Die Primer werden im Reaktionsansatz mit einer Endkonzentration von 0,1 - 3 µM vorgelegt und die Template-DNA im optimalen Fall mit 10² bis 10⁵ Kopien. Es können auch 10⁶ bis 10⁷ Kopien eingesetzt werden. Die entsprechende Polymerase wird dem Reaktionsansatz in einer Menge von 2-5 Units zugegeben. Ein typischer Reaktionsansatz hat ein Volumen von 20 - 100 µl.

Als weitere Zusätze können der Reaktion Rinderserumalbumin, Tween-20, Gelatin, Glycerin, Formamid oder DMSO beigesetzt werden (Dieffenbach und Dveksler, PCR Primer - A Laboratory Manual, Cold Spring Harbor Laboratory Press, USA 1995).

Ein typischer PCR-Verlauf besteht aus drei unterschiedlichen, sich aufeinanderfolgend wiederholenden Temperaturstufen. Vorab wird die Reaktion mit einer Temperaturerhöhung auf 92°C - 98°C für 4 bis 10 Minuten gestartet, um die vorgelegte DNA zu denaturieren. Dann folgen sich wiederholend zuerst ein Schritt zur Denaturierung der vorgelegten DNA von 10 - 60 Sekunden bei ungefähr 92-98°C, dann ein Schritt zum Binden der Primer an die vorgelegte DNA von 10 - 60 Sekunden bei einer bestimmten, von den Primern abhängigen Temperatur ("Annealing-Temperatur"), die erfahrungsgemäß bei 50°C bis 60°C liegt und sich für jedes Primerpaar einzeln berechnen läßt. Genaue Informationen dazu findet der Fachmann bei Rychlik et al. (Nucleic Acids Research 18 (21): 6409-6412). Abschließend folgt ein Synthese-Schritt zur Verlängerung der vorgelegten Primer ("Extension") bei dem jeweils für die Polymerase angegebenen Aktivitätsoptimum, üblicherweise je nach Polymerase im Bereich von 73°C bis 67°C bevorzugt 72°C bis 68°C. Die Dauer dieses Extensionschrittes hängt von der Leistungsfähigkeit der Polymerase und Länge des zu amplifizierenden PCR-Produktes ab. In einer typischen PCR dauert dieser Schritt 0,5 - 8 Minuten, bevorzugt 2 - 4 Minuten. Diese drei Schritte werden 30 bis 35 mal, gegebenenfalls bis zu 50 mal wiederholt. Ein abschließender "Extension"-Schritt von 4 - 10 Minuten beendet die Reaktion. Die auf diese Weise hergestellten Polynukleotide werden auch als Amplifikate bezeichnet; die Begriffe Nukleinsäurefragment und Nukleinsäuremolekül sind ebenfalls gebräuchlich.

Weitere Anleitungen und Informationen zur PCR findet der Fachmann beispielsweise im Handbuch "PCR-Strategies" (Innis, Felfand und Sninsky, Academic Press , Inc., 1995), im Handbuch von Diefenbach und Dveksler "PCR Primer - a laboratory manual" (Cold Spring Harbor Laboratory Press,1995), im Handbuch von Gait "Oligonukleotide synthesis: A Practical Approach" (IRL Press, Oxford, UK, 1984) und bei Newton und Graham "PCR" (Spektrum Akademischer Verlag, Heidelberg, Deutschland, 1994).

Die Nukleotidsequenz wird anschließend beispielsweise nach dem Kettenabbruchverfahren von Sanger et al. (Proceedings of the National Academies of Sciences, U.S.A., 74, 5463-5467 (1977)) mit den von Zimmermann et al. (Nucleic Acids Research 18, 1067pp (1990)) angegebenen Modifikationen bestimmt und das von dieser Nukleotidsequenz kodierte Polypeptid insbesondere bezüglich der Aminosäuresequenz analysiert. Dazu wird die Nukleotidsequenz in ein Programm zur Übersetzung von DNA-Sequenz in eine Aminosäuresequenz eingegeben. Geeignete Programme sind beispielsweise das Programm "Patentin", das bei Patentämtern, beispielsweise dem US-Patentamt (USPTO) erhältlich ist, oder das "Translate Tool", das auf dem ExPASy Proteomics Server im World Wide Web (Gasteiger et al., Nucleic Acids Research 31, 3784-3788 (2003)) verfügbar ist.

Auf diese Weise werden Mutanten identifiziert, deren mqo-Allele für Polypeptide mit Malat-Chinon-Oxidoreduktase Position jede proteinogene Aminosäure ausgenommen L-Serin enthalten. Bevorzugt wird der Austausch gegen L-Phenylalanin oder L-Alanin. Gegebenenfalls enthält die Aminosäuresequenz darüberhinaus einen Aminosäureaustausch L-Alanin gegen eine andere proteinogene Aminosäure, vorzugsweise L-Serin, an der Position 201 beziehungsweise an der entsprechenden oder vergleichbaren Position.

Erfindungsgemäße Mutanten eines coryneformen Bakteriums sind daher durch folgende Schritte erhältlich:
a) Behandlung eines coryneformen Bakteriums, das die Fähigkeit besitzt Aminosäuren auszuscheiden, mit einem mutagenen Agenz,
b) Isolierung und Vermehrung der in a) erzeugten Mutante,
c) Bestimmung der Fähigkeit der Mutante in einem Medium mindestens 0,5% mehr Aminosäure auszuscheiden oder im Zellinneren anzureichern als das in a) eingesetzte coryneforme Bakterium,
d) Bereitstellung von Nukleinsäure aus der in b) erhaltenen Mutante,
e) Herstellung eines Nukleinsäuremoleküls unter Verwendung der Polymerasekettenreaktion, der Nukleinsäure aus d), und eines Primerpaares bestehend aus einem ersten Primer umfassend mindestens 15 aufeinanderfolgende Nukleotide ausgewählt aus der Nukleotidsequenz zwischen Position 1 und 394 von SEQ ID NO:3 und einem zweitem Primer umfassend mindestens 15 aufeinanderfolgenden Nukleotide ausgewählt aus der komplementären Nukleotidsequenz zwischen Position 2002 und 1850 von SEQ ID NO:3.
f) Bestimmung der Nukleotidsequenz des in e) erhaltenen Nukleinsäuremoleküls, und Bestimmung der kodierten Aminosäuresequenz,
g) gegebenfalls Vergleich der in f) bestimmten. Aminosäuresesequenz mit SEQ ID NO:6, und
h) Identifizierung einer Mutante, die ein Polynukleotid enthält, das für ein Polypeptid kodiert, welches an Position 111 oder an vergleichbarer Position L-Phenylalanin und gegebenenfalls an Position 201 oder vergleichbarer Position L-Serin enthält.

Die auf diese Weise erzeugten Mutanten enthalten typischerweise eine (1) Kopie des beschriebenen gnd Allels.

In der SEQ ID NO:5 sind beispielhaft die Kodierregionen von mqo-Allelen erfindungsgemäßer Mutanten wiedergegeben. Die Kodierregion des Wildtypgens ist als SEQ ID NO:1 wiedergegeben. SEQ ID NO:1 enthält an Position 332 die Nukleobase Cytosin, an Position 331 die Nukleobase Thymin und an Position 601 die Nukleobase Guanin. SEQ ID NO:1 enthält an Position 331 bis 333 das für die Aminosäure L-Serin kodierende TCT Kodon und an Position 601 bis 603 das für die Aminosäure L-Alanin kodierende GCT Kodon. SEQ ID NO:5 enthält an Position 332 die Nukleobase Thymin. Durch diese Cytosin Thymin Transition entsteht an Position 331 bis 333 das für die Aminosäure L-Phenylalanin kodierende TTT Kodon. Durch Guanin-Thymin-Transversion entsteht an Position 601 bis 603 das für die Aminosäure L-Serin kodierende TCT Kodon. Darüber hinaus kann die in SEQ ID NO: 5 dargestellte Nukleotidsequenz weitere Basenaustausche enthalten, die sich durch die Mutagenesebehandlung ergeben haben, sich jedoch nicht in einer veränderten Aminosäuresequenz äußern. Derartige Mutationen werden in der Fachwelt auch als stille oder neutrale Mutationen bezeichnet. Diese stillen Mutationen können ebenfalls in dem für Mutagenesebehandlung eingesetztem coryneformen Bakterium bereits enthalten sein.

Die für die Mutagenese verwendeten coryneformen Bakterien besitzen bevorzugt bereits die Fähigkeit, die gewünschte Aminosäure in das sie umgebende Nährmedium beziehungsweise Fermentationsbrühe auszuscheiden oder im Zellinneren anzureichern.

L-Lysin produzierende coryneforme Bakterien besitzen typischerweise eine "feed back" resistente beziehungsweise desensibilisierte Aspartatkinase. Unter "feed back" resistenten Aspartatkinasen versteht man Aspartatkinasen, die im Vergleich zur Wildform eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese desensibilisierten Aspartatkinasen kodierenden Gene beziehungsweise Allele werden auch als lysC^{FBR}-Allele bezeichnet. Im Stand der Technik (Tabelle 1) sind zahlreiche lysC^{FBR}-Allele beschrieben, die für Aspartatkinase-Varianten kodieren, welche Aminosäureaustausche im Vergleich zum Wildtypprotein besitzen. Die Kodierregion des Wildtyp lysC-Gens von Corynebacterium glutamicum entsprechend der Zugangsnummer AX756575 der NCBI Datenbank ist in SEQ ID NO:21 und das von diesem Gen kodierte Protein in SEQ ID NO:22 dargestellt

**Tabelle 1**

| lysC^{FBR}-Allele kodierend für feed back resistente Aspartatkinasen | | | |
|---|---|---|---|
| Bezeichnung des Allels | Weitere Angaben | Referenz | Zugangsnummer |
| lysC^{FBR}-E05108 | | JP 1993184366-A (Sequenz 1) | E05108 |
| lysC^{FBR}-E06825 | lysC A279T | JP 1994062866-A (Sequenz 1) | E06825 |
| lysC^{FBR}-E06826 | lysC A279T | JP 1994062866-A (Sequenz 2) | E06826 |
| lysC^{FBR}-E06827 | | JP 1994062866-A (Sequenz 3) | E06827 |
| lysC^{FBR}-E08177 | | JP 1994261766-A (Sequenz 1) | E08177 |
| lysC^{FBR}-E08178 | lysC A279T | JP 1994261766-A (Sequenz 2) | E08178 |
| lysC^{FBR}-E08179 | lysC A279V | JP 1994261766-A (Sequenz 3) | E08179 |
| lysC^{FBR}-E08180 | lysC S301F | JP 1994261766-A (Sequenz 4) | E08180 |
| lysC^{FBR}-E08181 | lysC T308I | JP 1994261766-A (Sequenz 5) | E08181 |
| lysC^{FBR}-E08182 | | JP 1994261766-A (Sequenz 6) | E08182 |
| lysC^{FBR}-E12770 | | JP 1997070291-A (Sequenz 13) | E12770 |
| lysC^{FBR}-E14514 | | JP 1997322774-A (Sequenz 9) | E14514 |
| lysC^{FBR}-E16352 | | JP 1998165180-A (Sequenz 3) | E16352 |
| lysC^{FBR}-E16745 | | JP 1998215883-A (Sequenz 3) | E16745 |
| lysC^{FBR}-E16746 | | JP 1998215883-A (Sequenz 4) | E16746 |
| lysC^{FBR}I74588 | | US 5688671-A (Sequenz 1) | I74588 |
| lysC^{FBR}-I74589 | lysC A279T | US 5688671-A (Sequenz 2) | I74589 |
| lysC^{FBR}-I74590 | | US 5688671-A (Sequenz 7) | I74590 |
| lysC^{FBR}-I74591 | lysC A279T | US 5688671-A (Sequenz 8) | 174591 |
| lysC^{FBR}-I74592 | | US 5688671-A (Sequenz 9) | I74592 |
| lysC^{FBR}-I74593 | lysC A279T | US 5688671-A (Sequenz 10) | I74593 |
| lysC^{FBR}-I74594 | | US 5688671-A (Sequenz 11) | I74594 |
| lysC^{FBR}-I74595 | lysC A279T | US 5688671-A (Sequenz 12) | I74595 |
| lysC^{FBR}-I74596 | | US 5688671-A (Sequenz 13) | I74596 |
| lysC^{FBR}-I74597 | lysC A279T | US 5688671-A (Sequenz 14) | I74597 |
| lysC^{FBR}-X57226 | lysC S301Y | EP0387527 Kalinowski et al., Molecular and General Genetics 224:317-324 (1990) | X57226 |
| lysC^{FBR}-L16848 | lysC G345D | Follettie and Sinskey NCBI Nucleotide Database (1990) | L16848 |
| lysC^{FBR}-L27125 | lysC R320G | Jetten et al., Applied Microbiology Biotechnology 43:76-82 (1995) | L27125 |
| | lysC G345D | | |
| lysC^{FBR} | lysC T311I | WO0063388 (Sequenz 17) | |
| lysC^{FBR} | lysC S301F | US3732144 | |
| lysC^{FBR} | lysC S381F | EP0435132 | |
| lysC^{FBR} | lysC S317A | US5688671 (Sequenz 1) | |
| lysC^{FBR} | lysC T380I | WO 01/49854 | |

L-Lysin ausscheidende coryneforme Bakterien besitzen typischerweise einen oder mehrere der in Tabelle 1 aufgelisteten Aminosäureaustausche.

Bevorzugt werden folgende lysC^{FBR}-Allele: lysC A279T (Austausch von Alanin an Position 279 des kodierten Aspartatkinaseproteins gemäß SEQ ID NO: 22 gegen Threonin), lysC A279V (Austausch von Alanin an Position 279 des kodierten Aspartatkinaseproteins gemäß SEQ ID NO: 22 gegen Valin), lysC S301F (Austausch von Serin an Position 301 des kodierten Aspartatkinaseproteins gemäß SEQ ID NO: 22 gegen Phenylalanin), lysC T308I (Austausch von Threonin an Position 308 des kodierten Aspartatkinaseproteins gemäß SEQ ID NO: 22 gegen Isoleucin), lysC S301Y (Austausch von Serin an Position 308 des kodierten Aspartatkinaseproteins gemäß SEQ ID NO: 22 gegen Tyrosin), lysC G345D (Austausch von Glycin an Position 345 des kodierten Aspartatkinaseproteins gemäß SEQ ID NO: 22 gegen Asparaginsäure), lysC R320G (Austausch von Arginin an Position 320 des kodierten Aspartatkinaseproteins gemäß SEQ ID NO: 22 gegen Glycin), lysC T311I (Austausch von Threonin an Position 311 des kodierten Aspartatkinaseproteins gemäß SEQ ID NO: 22 gegen Isoleucin), lysC S381F (Austausch von Serin an Position 381 des kodierten Aspartatkinaseproteins gemäß SEQ ID NO: 22 gegen Phenylalanin), lysC S317A (Austausch von Serin an Position 317 des kodierten Aspartatkinaseproteins gemäß SEQ ID NO: 22 gegen Alanin) und lysC T380I (Austausch von Threonin an Position 380 des kodierten Aspartatkinaseproteins gemäß SEQ ID NO: 22 gegen Isoleucin).

Besonders bevorzugt werden das lysC^{FBR}-Allel lysC T311I (Austausch von Threonin an Position 311 des kodierten Aspartatkinaseproteins gemäß SEQ ID NO: 22 gegen Isoleucin) und ein lysC^{FBR}-Allel enthaltend mindestens einen Austausch ausgewählt aus der Gruppe A279T (Austausch von Alanin an Position 279 des kodierten Aspartatkinaseproteins gemäß SEQ ID NO: 22 gegen Threonin) und S317A (Austausch von Serin an Position 317 des kodierten Aspartatkinaseproteins gemäß SEQ ID NO: 22 gegen Alanin).

Das lysC^{FBR}-Allel lysC T311I ist in dem bei der DSMZ hinterlegten Stamm DM1797 enthalten. DM1797 ist eine Mutante von Corynebacterium glutamicum ATCC13032.

Nach der oben beschriebenen Art und Weise wurde, ausgehend von Stamm DM1797, eine als DM1808 bezeichnete Mutante isoliert, die ein mqo-Allel enthält, das für ein Polypeptid kodiert, bei dem an Position 111 der Aminosäuresequenz L-Phenylalanin enthalten ist. Die Nukleotidsequenz des mqo-Allels der Mutante DM1808 ist als SEQ ID NO:5 und die Aminosäuresequenz des kodierten Polypeptids als SEQ ID NO:6 dargestellt.

In der gleichen Weise wurde eine Mutante gefunden, die ein mqo-Allel enthält, das für ein Polypeptid kodiert bei dem an Position 111 der Aminosäuresequenz L-Alanin und bei dem an Position 201 der Aminosäuresequenz L-Serin enthalten ist. Die Nukleotidsequenz des mqo-Allels dieser Mutante ist als SEQ ID NO:9 und die Aminosäuresequenz des kodierten Polypeptids als SEQ ID NO:10 dargestellt.

Darüberhinaus können L-Lysin ausscheidende coryneforme Bakterien verwendet werden, die eine abgeschwächte Homoserin-Dehydrogenase oder Homoserinkinase aufweisen oder andere Eigenschaften besitzen, wie sie aus dem Stand der Technik bekannt sind.

L-Tryptophan produzierende coryneforme Bakterien besitzen typischerweise eine "feed back" resistente beziehungsweise desensibilisierte Anthranilatsynthase. Unter "feed back" resistenter Anthranilatsynthase versteht man Anthranilatsynthasen, die im Vergleich zur Wildform eine geringere Empfindlichkeit gegenüber der Hemmung (5 bis 10%, 10% bis 15% oder 10% bis 20%)durch Tryptophan oder 5-Fluorotryptophan (Matsui et al., Journal of Bacteriology 169 (11): 5330 - 5332(1987)) oder ähnliche Analoga. aufweisen. Die für diese desensibilisierten Anthranilatsynthasen kodierenden Gene beziehungsweise Allele werden auch als trpE^{FBR} -Allele bezeichnet. Beispiele für derartige Mutanten beziehungsweise Allele sind beispielsweise in der US 6180373 und EP0338474 beschrieben.

Die erhaltenen Mutanten zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Gegenstand der Erfindung ist ebenfalls ein isoliertes Polynukleotid ausgewählt aus einem der folgenden a) oder b)
a) ein Polynukleotid, das für ein Polypeptid mit Malat-Chinon-Oxidoreduktase Enzymaktivität und der Aminosäuresequenz SEQ ID NO: 2 kodiert, welches an Position 111 der Aminosäuresequenz von SEQ ID NO: 2 L-Phenylalanin und gegebenenfalls L-Serin an der Position 201 von SEQ ID NO: 2 besitzt,
b) ein Polynukleotid, das für ein Polypeptid mit Malat-Chinon-Oxidoreduktase Ezymaktivität kodiert, wobei das kodierte Polypeptid eine Aminosäuresequenz gemäß SEQ ID NO: 6 einschließlich eines bis fünf, bevorzugt höchstens zwei, höchstens drei oder höchstens vier, konservativer Aminosäureaustausch(e) umfasst, wobei in der Aminosäuresequenz an der Position, die der Position 111 der Aminosäuresequenz von SEQ ID NO: 6 entspricht, L-Phenylalanin enthalten ist,
wobei diese Polynukleotide die Aminosäuresekretion entsprechender Mutanten im Vergleich zum nicht mutagenisierten Ausgangsstamm mit der Aminosäuresequenz gemäß SEQ ID NO: 2 um mindestens 0,5 % erhöhen.

Das erfindungsgemäße Polynukleotid kann aus einer erfindungsgemäßen Mutante isoliert werden.

Weiterhin können für die Mutagenese des mqo-Gens in-vitro Methoden eingesetzt werden. Bei der Verwendung von in-vitro Methoden werden isolierte Polynukleotide, welche ein mqo-Gen eines coryneformen Bakteriums, vorzugsweise das im Stand der Technik beschriebene Wildtyp-Gen von Corynebacterium glutamicum, enthalten, einer mutagenen Behandlung unterzogen.

Bei den isolierten Polynukleotiden kann es sich beispielsweise um isolierte Gesamt-DNA beziehungsweise chromosomale DNA oder auch um Amplifikate des mqo-Gens handeln, die mit Hilfe der Polymerasekettenreaktion (PCR) hergestellt wurden. Derartige Amplifikate werden auch als PCR-Produkte bezeichnet; die Begriffe Nukleinsäuremolekül oder Nukleinsäurefragment sind ebenfalls gebräuchlich. Anleitungen zur Amplifikation von DNA-Sequenzen mit Hilfe der Polymerase-Kettenreaktion findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). Es ist ebenfalls möglich, das zu mutagenisierende mqo-Gen zunächst in einen Vektor, beispielsweise in einen Bakteriophagen oder in ein Plasmid einzubauen.

Geeignete Methoden für die in-vitro Mutagenese sind unter anderem die Behandlung mit Hydroxylamin nach Miller (Miller, J. H.: A Short Course in Bacterial Genetics. A Laboratory Manual and Handbook for Escherichia coli and Related Bacteria, Cold Spring Harbor Laboratory Press, Cold Spring Harbor, 1992), die Verwendung mutagener Oligonukleotide (T. A. Brown: Gentechnologie für Einsteiger, Spektrum Akademischer Verlag, Heidelberg, 1993 und R. M. Horton: PCR-Mediated Recombination and Mutagenesis, Molecular Biotechnology 3, 93-99 (1995)) und der Einsatz einer Polymerasekettenreaktion unter Verwendung einer DNA-Polymerase, die eine hohe Fehlerquote aufweist. Eine derartige DNA-Polymerase ist beispielsweise die Mutazyme DNA Polymerase (GeneMorph PCR Mutagenesis Kit, Nr.600550) der Firma Stratagene (LaJolla, CA, USA).

Weitere Anleitungen und Übersichten zur Erzeugung von Mutationen in-vivo oder in-vitro können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Ein erfindungsgemäßes isoliertes Polynukleotid, das für ein Polypeptid mit Malat-Chinon-Oxidoreduktase Enzymaktivität kodiert, umfasst vorzugsweise eine Aminosäuresequenz mit einer Länge von 500-Aminoäuren, wobei an Position 111 L-Phenylalanin enthalten ist.

Gegenstand der Erfindung ist weiterhin insbesondere ein isoliertes Polynukleotid, dass für ein Polypeptid mit Malat-Chinon-Oxidoreduktase Enzymaktivität kodiert, welches die Aminosäuresequenz von SEQ ID NO:6 umfasst.

Gegenstand der Erfindung ist weiterhin ein isoliertes Polynukleotid, dass für ein Polypeptid mit Malat-Chinon-Oxidoreduktase Enzymaktivität kodiert, welches die Aminosäuresequenz von SEQ ID NO:6 einschließlich einer Verlängerung am N- oder C-Terminus um mindestens eine (1) Aminosäure umfasst. Diese Verlängerung beträgt nicht mehr als 50, 40, 30, 20, 10, 5, 3 oder 2 Aminosäuren beziehungsweise Aminosäurereste.

Gegenstand der Erfindung ist weiterhin ein isoliertes Polynukleotid, das die Nukleotidsequenz gemäß SEQ ID NO:5 umfasst.

Gegenstand der Erfindung ist außerdem ein isoliertes Polynukleotid, welches ein Nukleinsäure-Molekül beziehungsweise DNA-Fragment umfasst, das für mindestens eine Aminosäuresequenz entsprechend Position 95 bis 127 von SEQ ID NO:2 oder das für mindestens eine Aminosäuresequenz entsprechend Position 79 bis 144 von SEQ ID NO: 2 oder das für mindestens eine Aminosäuresequenz entsprechend Position 62 bis 160 von SEQ ID NO: 2 oder das für mindestens eine Aminosäuresequenz entsprechend Position 45 bis 177 von SEQ ID NO: 2 oder das für mindestens eine Aminosäuresequenz entsprechend Position 27 bis 194 von SEQ ID NO: 2 oder das für mindestens eine Aminosäuresequenz entsprechend Position 11 bis 211 von SEQ ID NO: 2 oder das für mindestens eine Aminosäuresequenz entsprechend Position 2 bis 250 von SEQ ID NO: 2 oder das für mindestens eine Aminosäuresequenz entsprechend Position 2 bis 375 von SEQ ID NO: 2 oder das für mindestens eine Aminosäuresequenz entsprechend Position 2 bis 495 von SEQ ID NO: 2 kodiert, wobei an der Position entsprechend Position 111 von SEQ ID NO:2 L-Phenylalanin enthalten ist und wobei gegebenenfalls an der Position entsprechend Position 201 von SEQ ID NO: 2 L-Serin enthalten ist zur Verwendung zur Herstellung von coryneformen Bakterien, die eine im Vergleich zum nicht mutagenisierten Ausgangsstamm mit der Aminosäuresequenz gemäß SEQ ID NO: 2 um mindestens 0,5 % erhöhte Aminosäuresekretion aufweisen.

Ein Beispiel eines erfindungsgemäßen Leserasters umfassend ein Polynukleotid, das für mindestens die Aminosäuresequenz von Position 95 bis 127 entsprechend SEQ ID NO: 2 kodiert, wobei an der Position entsprechend Position 111 der Aminosäuresequenz L-Phenylalanin(Xaa steht für L-Phenylalanin) enthalten ist, ist nachfolgend aufgeführt:

Es ist ebenfalls als SEQ ID NO:15 dargestellt. Die von diesem Leseraster kodierte Aminosäuresequenz ist als SEQ ID NO:16 dargestellt, wobei Xaa für L-Phenylalanin steht.

Bevorzugt werden Nukleinsäuremoleküle, die für mindestens eine Aminosäuresequenz entsprechend Position 95 bis 127 von SEQ ID NO:6 oder mindestens entsprechend Position 79 bis 144 von SEQ ID NO: 6 oder mindestens entsprechend Position 62 bis 160 von SEQ ID NO: 6 oder mindestens entsprechend Position 45 bis 177 von SEQ ID NO: 6 oder mindestens entsprechend Position 27 bis 194 von SEQ ID NO: 6 oder mindestens entsprechend Position 11 bis 211 von SEQ ID NO: 6 oder mindestens entsprechend Position 2 bis 250 von SEQ ID NO: 6 oder mindestens entsprechend Position 2 bis 375 von SEQ ID NO: 6 oder mindestens entsprechend Position 2 bis 495 von SEQ ID NO: 6 kodieren.

Ein Beispiel eines erfindungsgemäßen Leserasters umfassend ein Polynukleotid, das für mindestens die Aminosäuresequenz entprechend Position 95 bis 127 von SEQ ID NO:6 kodiert, ist nachfolgend aufgeführt:

Das Leseraster ist ebenfalls als SEQ ID NO:17 dargestellt. SEQ ID NO:18 zeigt die von diesem Leseraster kodierte Aminosäuresequenz.

Ganz besonders bevorzugt werden Nukleinsäuremoleküle, die mindestens eine Nukleotidsequenz entsprechend Position 283 bis 381 von SEQ ID NO:5 oder mindestens eine Nukleotidsequenz entsprechend Position 235 bis 432 von SEQ ID NO:5 oder mindestens eine Nukleotidsequenz entsprechend Position 184 bis 480 von SEQ ID NO: SEQ ID NO:5 oder mindestens eine Nukleotidsequenz entsprechend Position 133 bis 531 von SEQ ID NO:5 oder mindestens eine Nukleotidsequenz entsprechend Position 85 bis 582 von SEQ ID NO:5 oder mindestens eine Nukleotidsequenz entsprechend Position 28 bis 630 von SEQ ID NO:5 oder mindestens eine Nukleotidsequenz entsprechend Position 4 bis 753 von SEQ ID NO:5 oder mindestens eine Nukleotidsequenz entsprechend Position 4 bis 1125 von SEQ ID NO:5 oder mindestens eine Nukleotidsequenz entsprechend Position 4 bis 1485 von SEQ ID NO:5 umfassen.

Die erfindungsgemäßen, isolierten Polynukleotide können dazu verwendet werden, um rekombinante Stämme von Mikroorganismen herzustellen, die im Vergleich zum Ausgangs- beziehungsweise Elternstamm in verbesserter Weise Aminosäuren in das sie umgebende Medium abgeben oder im Zellinneren anhäufen.

Eine verbreitete Methode zum Einbau von Mutationen in Gene coryneformer Bakterien ist die des Allelaustausches, die auch unter der Bezeichnung "gene replacement" bekannt ist. Bei diesem Verfahren wird ein DNA-Fragment, welches die interessierende Mutation enthält, in den gewünschten Stamm eines coryneformen Bakteriums überführt und die Mutation durch wenigstens zwei Rekombinationsereignisse beziehungsweise "cross over"-Ereignisse in das Chromosom des gewünschten Stammes inkorporiert beziehungsweise die im betreffenden Stamm vorhandene Sequenz eines Gens gegen die mutierte Sequenz ausgetauscht.

Von Schwarzer und Pühler (Bio/Technology 9, 84-87 (1991) wurde diese Methode verwendet um ein lysA-Allel, welches eine Deletion trug, und um ein lysA-Allel, welches eine Insertion trug in das Chromosom von C. glutamicum anstelle des Wildtypgens einzubauen. Von Schäfer et al. (Gene 145, 69-73 (1994)) wurde diese Methode eingesetzt um eine Deletion in das hom-thrB Operon von C. glutamicum einzubauen. Von Nakagawa et al. (EP 1108790) wurde diese Methode eingesetzt um verschiedene Mutationen ausgehend von den isolierten Allelen in das Chromosom von C. glutamicum einzubauen. Auf diese Weise gelang es Nakagawa et al. eine als Val59Ala bezeichnete Mutation in das Homoserin-Dehydrogenase Gen (hom), eine als Thr311Ile bezeichnete Mutation in das Aspartatkinase-Gen (lysC bzw. ask), eine als Pro458Ser bezeichnete Mutation in das Pyruvat-Carboxylase-Gen (pyc) und eine als Ala213Thr bezeichnete Mutation in das Glucose-6-phoshat-Dehydrogenase Gen (zwf) von C. glutamicum Stämmen einzubauen.

Für ein erfindungsgemäßes Verfahren kann ein erfindungsgemäßes Polynukleotid verwendet werden, welches die gesamte Kodierregion umfasst, wie beispielsweise in SEQ ID NO:5 gezeigt, oder welches einen Teil der Kodierregion umfasst, wie beispielsweise die Nukleotidsequenz, die für mindestens die Aminosäuresequenz entsprechend Position 95 bis 127 von SEQ ID NO:6 kodiert und die als SEQ ID NO:15 und 17 dargestellt sind. Der Teil der Kodierregion entsprechend SEQ ID NO: 15 und 17 hat eine Länge von 99 Nukleobasen. Bevorzugt werden solche Teile der Kodierregion, deren Länge ≥ 195 Nukleobasen beträgt, wie beispielsweise Nukleinsäuremoleküle, die für mindestens eine Aminosäuresequenz entsprechend Position von 79 bis 144 von SEQ ID NO: 6 kodieren. Ganz besonders bevorzugt werden solche Teile der Kodierregion, deren Länge ≥ 295 Nukleobasen beträgt, wie beispielsweise Nukleinsäuremoleküle, die für mindestens eine Aminosäuresequenz entsprechend Position von 62 bis 160 von SEQ ID NO: 6 kodieren

Das DNA-Fragment enthaltend die interessierende Mutation liegt bei dieser Methode typischerweise in einem Vektor, insbesondere einem Plasmid, vor, das vorzugsweise von dem mit der Mutation zu versehenden Stamm nicht oder nur begrenzt repliziert wird. Als Hilfs- oder Zwischenwirt, in dem der Vektor replizierbar ist, wird im Allgemeinen ein Bakterium der Gattung Escherichia bevorzugt der Spezies Escherichia coli verwendet.

Beispiele für derartige Plasmidvektoren sind die von Schäfer et al. (Gene 145, 69-73 (1994)) beschriebenen pK*mob und pK*mobsacB Vektoren, wie beispielsweise pK18mobsacB, und die in der WO 02/070685 und WO 03/014362 beschriebenen Vektoren. Diese sind in Escherichia coli aber nicht in coryneformen Bakterien replikativ. Besonders geignet sind Vektoren, die ein konditional negativ dominant wirkendes Gen wie beispielsweise das sacB-Gen (Levansucrase-Gen) von beispielsweise Bacillus oder das galK-Gen (Galaktosekinase) von beispielsweise Escherichia coli enthalten. (Unter einem konditional negativ dominant wirkenden Gen versteht man ein Gen, das unter bestimmten Bedingungen nachteilig beispielsweise toxisch für den Wirt ist, unter anderen Bedingungen aber keine negativen Auswirkungen auf den das Gen tragenden Wirt hat.) Diese ermöglichen die Selektion auf Rekombinationsereignisse, bei denen der Vektor aus dem Chromosom eliminiert wird. Weiterhin wurde von Nakamura et al. (US-A-6,303,383) ein Temperatur-sensitives Plasmid für coryneforme Bakterien beschrieben, das lediglich bei Temperaturen unterhalb von 31°C replizieren kann.

Der Vektor wird anschließend durch Konjugation beispielsweise nach der Methode von Schäfer (Journal of Bacteriology 172, 1663-1666 (1990)) oder Transformation beispielsweise nach der Methode von Dunican und Shivnan (Bio/Technology 7, 1067-1070 (1989)) oder der Methode von Thierbach et al. (Applied Microbiology and Biotechnology 29, 356-362 (1988)) in das coryneforme Bakterium überführt. Gegebenenfalls kann die Überführung der DNA auch durch Partikelbeschuss erzielt werden.

Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation und erhält ein rekombinantes Bakterium.

Zur Identifizierung und Charakterisierung der erhaltenen Stämme können unter anderem die Methoden der Southern Blotting Hybridisierung, der Polymerase-Kettenreaktion, der Sequenzbestimmung, die Methode des "Fluorescence Resonance Energy Transfer" (FRET) (Lay et al. Clinical Chemistry 43, 2262-2267 (1997)) oder Methoden der Enzymologie eingesetzt werden.

Ein weiterer Gegenstand der Erfindung ist dementsprechend ein Verfahren zur Herstellung eines coryneformen Bakteriums, bei dem man
a) ein erfindungsgemäßes Polynukleotid in ein coryneformes Bakterium überführt,
b) das im Chromosom des coryneformen Bakteriums vorhandene Malat-Chinon-Oxidoreduktase Gen, das für eine Aminosäuresequenz mit L-Serin an Position 111 oder vergleichbaren Position der Aminosäuresequenz kodiert, gegen das Polynukleotid aus a) austauscht, das für eine Aminosäuresequenz kodiert, die an der Position 111 oder einer vergleichbaren Position der Aminosäuresequenz eine andere L-Aminosäure, vorzugsweise L-Phenylalanin oder L-Alanin, und gegebenenfalls an Position 201 jede proteinogene Aminosäure ausgenommen L-Alanin, bevorzugt die Aminosäure L-Serin, besitzt, und
c) das gemäß Schritt a) und b) erhaltene coryneforme Bakterium vermehrt.

Auf diese Weise erhält man ein rekombinantes coryneformes Bakterium, welches anstelle des Wildtyp mqo-Gens ein erfindungsgemäßes mqo-Allel enthält.

Weitere Gegenstände der Erfindung sind dementsprechend Wirte beziehungsweise Wirtszellen, bevorzugt Mikroorganismen, besonders bevorzugt coryneforme Bakterien und Bakterien der Gattung Escherichia, die die erfindungsgemäßen Polynukleotide enthalten. Gegenstand der Erfindung sind gleichfalls Mikrorganismen, die unter Verwendung der isolierten Polynukleotide hergestellt wurden. Derartige Mikroorganismen oder Bakterien werden auch als rekombinante Mikroorganismen oder rekombinante Bakterien bezeichnet. In gleicher Weise sind Vektoren, die die erfindungsgemäßen Polynukleotide enthalten, Gegenstand der Erfindung. Schließlich sind Wirte, die diese Vektoren enthalten, ebenfalls Gegenstand der Erfindung.

Die erfindungsgemäßen, isolierten Polynukleotide können gegebenenfalls zur Erzielung einer Überexpression der von ihnen kodierten Proteine/Polypeptide verwendet werden. Unter Überexpression versteht man allgemein eine Erhöhung der intrazellulären Konzentration oder Aktivität einer Ribonukleinsäure, eines Proteins oder eines Enzyms. Im Falle der vorliegenden Erfindung werden mqo-Allele beziehungsweise Polynukleotide überexprimiert, die für Malat-Chinon-Oxidoreduktasen kodieren, die an Position 111 der Aminosäuresequenz des kodierten Polypeptids jede proteinogene Aminosäure ausgenommen L-Serin enthalten, wobei der Austausch gegen L-Phenylalanin oder L-Alanin bevorzugt wird. Gegebenenfalls enthält das kodierte Protein außerdem einen Austausch von L-Alanin gegen eine andere proteinogene Aminosäure, bevorzugt L-Serin an Position 201 der Aminosäuresequenz.

Es ist bekannt, dass durch wirtseigene Enzyme - sogenannte Aminopeptidasen - N-terminale Aminosäuren, insbesondere das N-terminale Methionin, vom gebildeten Polypeptid abgespalten werden können.

Die genannte Erhöhung der Konzentration oder Aktivität eines Genproduktes lässt sich beispielsweise dadurch erzielen, dass man die Kopienzahl der entsprechenden Polynukleotide um mindestens eine Kopie erhöht.

Eine weit verbreitete Methode zur Erhöhung der Kopienzahl besteht darin, dass man das entsprechende Gen oder Allel in einen Vektor, bevorzugt ein Plasmid, einbaut, der von einem coryneformen Bakterium repliziert wird. Geeignete Plasmidvektoren sind beispielsweise pZ1 (Menkel et al., Applied and Environmental Microbiology (1989) 64: 549-554) oder die von Tauch et al. (Journal of Biotechnology 99, 79-91 (2002)) beschriebenen pSELF-Vektoren. Ein Übersichtsartikel zum Thema Plasmide in Corynebacterium glutamicum findet sich bei Tauch et al. (Journal of Biotechnology 104, 27-40 (2003)).

Eine andere verbreitete Methode zur Erzielung einer Überexpression ist das Verfahren der chromosomalen Genamplifikation. Bei dieser Methode wird mindestens eine zusätzliche Kopie des interessierenden Gens oder Allels in das Chromosom eines coryneformen Bakteriums eingefügt.

In einer Ausführungsform, wie sie beispielsweise bei Reinscheid et al. (Applied and Environmental Microbiology 60, 126-132) für das hom-thrB-Operon beschrieben ist, wird ein in C. glutamicum nicht-replikatives Plasmid, welches das interessierende Gen enthält,in ein coryneformes Bakterium überführt. Nach homologer Rekombination mittels eines "cross over"-Ereignisses enthält der resultierende Stamm mindestens zwei Kopien des betreffenden Gens beziehungsweise Allels.

In einer anderen Ausführungsform, die in der WO 03/040373 und US-2003-0219881-A1 beschrieben ist, wird eine oder mehrere Kopie(n) des interessierenden Gens mittels mindestens zweier Rekombinationsereignisse in einen gewünschten Ort des Chromosoms von C. glutamicum eingefügt. Auf diese Weise wurde beispielsweise eine Kopie eins lysC-Allels, das für eine L-Lysin insensitive Aspartatkinase kodiert, in das gluB-Gen von C. glutamicum eingebaut.

In einer weiteren Ausführungsform, die in der WO 03/014330 und US-2004-0043458-A1 beschrieben ist, wird mittels mindestens zweier Rekombinationsereignisse am natürlichen Ort mindestens eine weitere Kopie, vorzugsweise in tandem-Anordnung zu dem bereits vorhandenen Gen oder Allel, des interessierenden Gens eingebaut. Auf diese Weise wurde beispielsweise eine tandem-Duplikation eines lysC^{FBR}-Allels am natürlichen lysC-Genort erzielt.

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für Corynebacterium glutamicum sind beispielsweise in dem Übersichtsartikel von Patek et al. (Journal of Biotechnology 104(1-3), 311-323 (2003) beschrieben. Weiterhin können die hinlänglich bekannten von Amann et al. (Gene 69(2), 301-315 (1988)) und Amann und Brosius (Gene 40(2-3), 183-190 (1985)) beschriebenen Promotoren T3, T7, SP6, M13, lac, tac und trc verwendet werden. Ein derartiger Promotor kann beispielsweise stromaufwärts des mqo-Allels, typischerweise im Abstand von ungefähr 1 - 500 Nukleotiden vom Startkodon, eines rekombinanten coryneformen Bakteriums eingefügt werden, welches anstelle der an Position 111 natürlicherweise vorhanden Aminosäure L-Serin eine andere proteinogene Aminsäure enthält. Ein derartiger Promotor kann naturgemäß ebenfalls stromaufwärts des mqo-Allels einer erfindungsgemäßen Mutante eingefügt werden. Es ist weiterhin möglich ein erfindungsgemäßes isoliertes Polynukleotid, das für eine erfindungsgemäße Variante der Malat-Chinon-Oxidoreduktase kodiert, mit einem Promotor zu verknüpfen und die erhaltene Expressionseinheit in ein extrachromosomal replizierendes Plasmid oder in das Chromosom eines coryneformen Bakteriums einzubauen.

Darüberhinaus kann die Promotor- und Regulationsregion oder die Ribosomenbindungsstelle, die sich stromaufwärts des Strukturgens befindet, mutiert werden. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Alternativ kann weiterhin eine Überexpression des betreffenden Gens oder Allels durch Veränderung der Medienzusammensetzung und Kulturführung erreicht werden.

Durch die Maßnahmen der Überexpression wird die Aktivität oder Konzentration des entsprechenden Proteins/Polypeptids im allgemeinen um mindestens 10%, 25%, 50%, 75%, 100%, 150%, 200%, 300%, 400% oder 500%, maximal bis 1000% oder 2000%, bezogen auf die Aktivität oder Konzentration des Proteins im Ausgangs-Mikroorganismus beziehungsweise Elternstammes erhöht. Unter einem Ausgangs-Mikroorganismus oder Elternstamm versteht man einen Mikroorganismus, an dem die Massnahmen der Erfindung durchgeführt werden.

Eine Methode zur Bestimmung der enzymatischen Aktivität der Malat-Chinon-Oxidoreduktase ist bei Molenaar et al. (Journal of Bacteriology 182(24), 6884-6891 (2000)) beschrieben.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 111: 2630-2647 (1999)) bestimmt werden.

Gegenstand der Erfindung sind dementsprechend Verfahren zur Überexpression der erfindungsgemäßen Malat-Chinon-Oxidoreduktasen. Ein erfindungsgemässes Verfahren zur Überexpression besteht unter anderem darin, dass man die Kopienzahl eines erfindungsgemässen Polynukleotids, das für eine Malat-Chinon-Oxidoreduktase Variante kodiert, bei der an Position 111 oder entsprechenden Position der kodierten Aminosäuresequenz L-Phenylalanin enthalten ist, um mindestens eine (1) oder um mehrere Kopien erhöht. Ein weiteres erfindungsgemässes Verfahren besteht darin, dass man einen Promotor funktionell mit dem Polynukleotid verknüpft.

Gegenstand der Erfindung sind weiterhin Mikroorganismen, die eine erhöhte Konzentration oder Aktivität der erfindungsgemäßen Malat-Chinon-Oxidoreduktase Varianten in ihrem Zellinneren aufweisen.

Zusätzlich kann es für die verbesserte Produktion von L-Aminosäuren vorteilhaft sein, in den erfindungsgemäßen Mutanten oder rekombinanten Stämme eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine überzuexprimieren. Die Verwendung endogener Gene wird im allgemeinen bevorzugt.

Unter "endogenen Genen" oder "endogenen Nukleotidsequenzen" versteht man die in der Population einer Art vorhandenen Gene beziehungsweise Nukleotidsequenzen oder Allele.

So kann für die Herstellung von L-Lysin eines oder mehrere der Gene, ausgewählt aus der Gruppe
- ein für ein Dihydrodipicolinat-Synthase kodierendes Gen dapA, wie beispielsweise das in der EP 0 197 335 beschriebene dapA-Gen des Wildtyps von Corynebacterium glutamicum,
- ein für eine Glucose-6-Phosphat Dehydrogenase kodierendes Gen zwf, wie beispielsweise das in der JP-A-09224661 und EP-A-1108790 beschriebene zwf-Gen des Wildtyps von Corynebacterium glutamicum,
- die in der US-2003-0175911-A1 beschriebenen zwf-Allele von Corynebacterium glutamicum, die für ein Protein kodieren, bei dem beispielsweise das L-Alanin an Position 243 der Aminosäuresequenz durch L-Threonin ersetzt ist oder bei dem die L-Asparaginsäure an Position 245 durch L-Serin ersetzt ist,

- ein für eine Pyruvat-Carboxylase kodierendes Gen pyc, wie beispielsweise das in der DE-A-198 31 609 und EP 1108790 beschriebene pyc-Gen des Wildtyps von Corynbebacterium glutamicum,
- das für das in der EP 1 108 790 beschriebene pyc-Allel von Corynebacterium glutamicum, das für ein Protein kodiert, bei dem L-Prolin an Position 458 der Aminosäuresequenz durch L-Serin ersetzt ist,
- die in der WO 02/31158 beschriebene pyc-Allele von Corynebacterium glutamicum, die für Proteine kodieren, welche gemäß Aspruch 1 einen oder mehrere der Aminosäureaustausche ausgewählt aus der Gruppe L-Glutaminsäure an Position 153 ersetzt durch L-Asparaginsäure, L-Alanin an Position 182 ersetzt durch L-Serin, L-Alanin an Position 206 ersetzt durch L-Serin, L-Histidin an Position 227 ersetzt durch L-Arginin, L-Alanin an Position 452 ersetzt durch Glycin und L-Asparaginsäure an Position 1120 ersetzt durch L-Glutaminsäure tragen (Figur 2A von WO 02/31158 gibt zwei verschiedene Startpositionen für die Pyruvat-Carboxylase an, die sich um eine Länge entsprechend 17 Aminosäuren unterscheiden. Dementsprechend entspricht die Position 153 nach Anspruch 1 von WO 02/31158 der Position 170 von Fig.2A von WO 02/31158, die Position 182 nach Anspruch 1 der Position 199 von Fig.2A, die Position 206 nach Anspruch 1 der Position 223 von Fig. 2A, die Position 227 nach Anspruch 1 der Position 244 von Fig.2A, die Position 452 nach Anspruch 1 der Position 469 von Fig. 2A, die Position 1120 nach Anspruch 1 der Position 1137 von Fig.2B. In Figur 2A von WO 02/31158 ist weiterhin ein Aminosäureaustausch A (Alanin) gegen G (Glycin) an Position 472 angegeben. Die Position 472 des Proteins mit der N terminalen Sequenz MTA entspricht der Position 455 des Proteins mit der N-terminalen Sequenz MST gemäß Fig. 2A. In Fig. 2B von WO 02/31158 ist weiterhin ein Aminosäureaustausch D (Asparaginsäure) gegen E (Glutaminsäure) an Position 1133 des Proteins mit dem N-Terminus MTA angegeben.),

- ein für eine Aspartatkinase kodierendes lysC Gen wie beispielsweise das als SEQ ID NO:281 in der EP-A-1108790 (Siehe auch Zugangsnummer AX120085 und 120365) und das als SEQ ID NO:25 in der WO 01/00843 (Siehe Zugangsnummer AX063743) beschriebene von lysC-Gen des Wildtyps von Corynebacterium glutamicum,
- ein für eine feed-back resistente Aspartatkinase Variante kodierendes lysC^{FBR} Allel, insbesondere entsprechend Tabelle 1,
- ein für ein Lysin-Export-Protein kodierendes Gen lysE, wie beispielsweise das in der DE-A-195 48 222 beschriebene lysE-Gen von des Wildtyps Corynebacterium glutamicum,
- das für das Zwal-Protein kodierende Gen zwa1 des Wildtyps
   von Corynebacterium glutamicum (US 6,632,644) überexprimiert werden.

Weiterhin kann es für die Produktion von L-Lysin vorteilhaft sein, neben der Verwendung der erfindungsgemäßen Allele des mqo-Gens gleichzeitig eines oder mehrere der endogenen Gene, ausgewählt aus der Gruppe
- ein für die Glucose-6-Phosphat-Isomerase kodierendes Gen pgi, wie beispielsweise das in der US 6,586,214 und US 6,465,238 beschriebene pgi-Gen von Corynebacterium glutamicum,
- ein für die Homoserin-Dehydrogenase kodierendes Gen hom, wie beispielsweise das in der EP-A-0131171 beschriebene hom-Gen von Corynebacterium glutamicum,
- ein für die Homoserin-Kinase kodierendes Gen thrB, wie beispielsweise das von Peoples et al. (Molecular Microbiology 2 (1988): 63 - 72)) beschriebene thrB-Gen von Corynebacterium glutamicum und
- ein für die Phosphofruktokinase kodierendes Gen pfkB, wie beispielsweise das in der WO 01/00844 (Sequenz Nr. 57) beschriebene pfkB-Gen von Corynebacterium glutamicum,
abzuschwächen oder auszuschalten.

Die aufgeführten Abschwächungsmaßnahmen können gegebenenfalls mit den aufgeführten zusätzlichen Überexpressionsmaßnahmen (Überexpression des dapA-Gens, des zwf-Gens etc.) kombiniert werden.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Als Mutationen zur Erzeugung einer Abschwächung kommen Transitionen, Transversionen, Insertionen und Deletionen von mindestens einem (1) Basenpaar bzw. Nukleotid in Betracht. In Abhängigkeit von der Wirkung des durch die Mutation hervorgerufenen Aminosäureaustausches auf die Enzymaktivität wird von Fehlsinnmutationen ("missense mutations") oder Nichtsinnmutationen ("nonsense mutations") gesprochen. Die Fehlsinnmutation führt zu einem Austausch einer gegebenen Aminosäure in einem Protein gegen eine andere, wobei es sich insbesondere um einen nichtkonservative Aminosäureaustausch handelt. Hierdurch wird die Funktionsfähigkeit bzw. Aktivität des Proteins beeinträchtigt und auf einen Wert von 0 bis 75%, 0 bis 50%, 0 bis 25%, 0 bis 10% oder 0 bis 5% reduziert. Die Nichtsinnmutation führt zu einem Stop-Kodon im Kodierbereich des Gens und damit zu einem vorzeitigen Abbruch der Translation. Insertionen oder Deletionen von mindestens einem Basenpaar in einem Gen führen zu Rasterverschiebungsmutationen ("frame shift mutations"), die dazu führen, dass falsche Aminosäuren eingebaut werden oder die Translation vorzeitig abbricht. Entsteht als Folge der Mutation ein Stop-Kodon im Kodierbereich, so führt dies ebenfalls zu einem vorzeitigen Abbruch der Translation. Deletionen von mindestens einem (1) oder mehreren Kodonen führen typischerweise ebenfalls zu einem vollständigen Ausfall der Enzymaktivität.

Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden. Weitere Maßnahmen sind im Stand der Technik beschrieben.

Die durch die Massnahmen der Erfindung erhaltenen isolierten coryneformen Bakterien zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Unter isolierten Bakterien sind die erfindungsgemäßen, isolierten beziehungsweise erzeugten Mutanten und rekombinanten Bakterien, insbesonders coryneformer Bakterien, zu verstehen, welche ein mqo-Allel enthalten, das für eine Malat-Chinon-Oxidoreduktase kodiert, die den beschriebenen Aminosäureaustausch an Position 111 der Aminosäuresequenz und gegebenenfalls einen Aminosäureaustausch L-Alanin gegen eine andere proteinogene Aminosäure, bevorzugt L-Serin, an Position 201 enthält.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Die erfindungsgemäßen, isolierten coryneformen Bakterien können kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5,605,818, in der US 5,275,940 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Anionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Gegenstand der Erfindung ist dementsprechend ein Verfahren zur Herstellung einer L-Aminosäure bei dem man
a) ein isoliertes coryneformes Bakterium in einem geeignetem Medium fermentiert, wobei das Bakterium ein für ein Polypeptid mit Malat-Chinon-Oxidoreduktase Enzymaktivität kodierendes erfindungsgemäßes Gen enthält, wobei in den Aminosäuresequenzen des Polypeptids das L-Serin an der Position 111 oder der entsprechenden Position durch L-Phenylalanin ersetzt ist und wobei gegebenenfalls das L-Alanin an Position 201 oder der entsprechenden Position gegen eine andere proteinogene Aminosäure, bevorzugt L-Serin, ersetzt ist, und
b) die L-Aminosäure in der Fermentationsbrühe oder in den Zellen des isolierten coryneformen Bakteriums anreichert.

Die auf diese Weise hergestellte Fermentationsbrühe wird anschließend zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

Im Falle der Aminosäure L-Lysin sind im Stand der Technik im wesentlichen vier verschiedene Produktformen bekannt.

Eine Gruppe L-Lysin haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen die L-Aminosäure aus der Fermentationsbrühe gesammelt, isoliert oder gereinigt wird, um das L-Aminosäure haltige Produkt oder die gereinigte L-Aminosäure herzustellen.

Zur Herstellung von festen, reinen L-Aminosäuren werden im wesentlichen Methoden der Ionenaustauschchromatographie gegebenfalls unter Verwendung von Aktivkohle und Methoden der Kristallisierung angewendet. Im Falle des Lysin erhält man auf diese Weise die entsprechende Base oder ein entsprechendes Salz wie beispielsweise das Monohydrochlorid (Lys-HCl) oder das Lysinsulfat (Lys₂-H₂SO₄).

Im Falle des Lysin ist in der EP-B-0534865 ein Verfahren zur Herstellung wässriger, basischer L-Lysin haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung der erfindungsgemäßen Bakterien werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

Die chemischen Bestandteile der Biomasse sind unter anderem die Zellhülle, beispielsweise das Peptidoglykan und das Arabinogalaktan, das Protein bzw. Polypeptid, beispielsweise das Malat-Chinon-Oxidoreduktase Polypeptid, Lipide und Phospholipide und Nukleinsäuren (DNA und RNA), beispielsweise Polynukleotide enthaltend die erfindungsgemäße Mutation. Als Folge der Massnahmen der Inaktivierung und/oder der weitereren Verfahrensschritte (beispielsweise Ansäuerung,Sprühtrocknung, Granulation etc.) liegen Nukleinsäuren typischerweise als Fragmente mit eine Länge von unter anderem ≥40 - 60 bp, >60 - 80 bp, >80 - 100 bp, >100 - 200 bp, >200 - 300 bp, >300 - 400 bp, >400 - 500 bp, >500 - 750 bp, >750 - 1000 bp, >1000 -1250 bp, >1250 - 1500 bp, >1500 - 1750 bp, >1750 - 2000 bp, >2000 - 2500 bp, >2500 - 3000 bp, >3000 - 4000 bp, >4000 - 5000 bp vor.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert gestellt werden (GB 1,439,728 oder EP 1 331 220). Es ist gleichfalls möglich die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig das heißt > 95% oder > 98% im Produkt. In diesem Sinne bedeutet der Begriff "Fermentationsbrühebasis", dass ein Produkt mindestens einen Teil der Bestandteile der Fermentationsbrühe enthält.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt d. h. ohne vorherige Aufkonzentrierung durch Sprühtrocknung oder Sprühgranulation zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 pm Durchmesser gemeint.

Mit "grobkörnig" ist ein Produkt mit einem überwiegendem Anteil (> 50 %) einer Korngröße von 200 bis 2000 pm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerbares und weitgehend staubfreies Produkt überführt werden.

Der Begriff "staubfrei" bedeutet, daß das Produkt lediglich geringe Anteile (< 5 %) an Körngrößen unter 100 pm Durchmesser enthält.

"Lagerbar", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann ohne das ein wesentlicher Verlust (< 5%) der jeweiligen Aminosäure auftritt.

Ein weiterer Gegenstand der Erfindung ist dementsprechend ein Verfahren zur Herstellung eines L-Aminosäure, bevorzugt L-Lysin oder L-Tryptophan, haltigen Produktes, bevorzugt Tierfuttermittel-Additivs, aus Fermentationsbrühen, gekennzeichnet durch die Schritte
a) Kultivierung und Fermentation eines erfindungsgemäen L-Aminosäure ausscheidenden coryneformen Bakteriums, welches mindestens ein erfindungsgemäßes mqo-Allel enthält, das für ein Polypeptid mit Malat-Chinon-Oxidoreduktase Aktivität kodiert, welches eine Aminosäuresequenz umfasst, bei der an Position 111 oder der vergleichbaren Position L-Phenylalanin enthalten ist, wobei gegebenenfalls an Position 201 oder der vergleichbaren Position L-Serin enthalten ist, in einem Fermentationsmedium,
b) Entfernung der während der Fermentation gebildeten Biomasse in einer Menge von 0 bis 100 Gew.-%, und
c) Trocknung der gemäß a) und/oder b) erhaltenen Fermentationsbrühe, um das Produkt in der gewünschten Pulver- oder Granulatform zu erhalten
wobei gegebenenfalls vor Schritt b) oder c) eine Säure ausgewählt aus der Gruppe Schwefelsäure, Phosphorsäure oder Salzsäure hinzugefügt wird.

Vorzugsweise wird im Anschluss an Schritt a) oder b) Wasser aus der L-Aminosäure haltigen Fermentationsbrühe entfernt (Aufkonzentration).

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) Stearaten.

Weiterhin ist es vorteilhaft die Oberfläche der erhaltenen Granulate mit Ölen zu versehen so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethycellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 pm Durchmesser. Der Anteil an Staub d. h. Partikeln mit einer Korngrösse < 100 µm liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonate, Kieselsäuren, Silikate, Alginate, Stearate, Stärken, Gummis und Celluloseether, wie in der DE-C-4100920 beschrieben, in einen Zustand gebracht werden, in dem es stabil gegenüber der Verdauung durch Tiermägen insbesondere dem Magen von Wiederkäuern ist.

Zur Einstellung einer gewünschten Aminosäurekonzentration im Produkt kann je nach Anforderung die entsprechende Aminosäure während des Verfahrens in Form eines Konzentrates oder gebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses hinzugefügt werden.

Im Falle des Lysin wird bei der Herstellung Lysin-haltiger Produkte das Verhältnis der Ionen so eingestellt, dass das Ionenverhältnis entsprechend nachstehender Formel 2x[SO₄²⁻]+[Cl⁻]-[NH₄⁺]-[Na⁺]-[K⁺]-2x[Mg⁺]-2x[Ca²⁺]/[L-Lys] 0,68 bis 0,95, bevorzugt 0,68 bis 0,90 ergibt so wie von Kushiki et al. in der US 20030152633 beschrieben.

Im Falle des Lysin hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-%, 72 Gew.-%, 73 Gew.-% sind ebenfalls möglich.

Im Falle einer elektrisch neutralen Aminosäure wie dem L-Tryptophan hat das auf diese Weise hergestellte feste Produkt auf Fermentationsbrühebasis einen Aminosäuregehalt von mindestens 5 Gew.-%, 10 Gew.-%, 20 Gew.-%, 30 Gew.-% und maximal 50 Gew.-%, 60 Gew.-%, 70 Gew.-%, 80 Gew.-%, 90 Gew.-% oder bis 95 Gew.-%.

Der Wassergehalt des festen Produktes beträgt bis zu 5 Gew.-%, bevorzugt bis zu 4 Gew.-%, und besonders bevorzugt weniger als 3 Gew.-%.

Gegenstand der Erfindung ist daher auch ein L-Lysin haltiges Futtermitteladditiv auf Fermentationsbrühebasis, welches folgende Merkmale aufweißt
a) einen Lysingehalt (als Base) von mindestens 10 Gew.-% bis maximal 73 Gew.-%,
b) einen Wassergehalt von höchstens 5 Gew.-%, und
c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Gegenstand der Erfindung ist weiterhin auch ein L-Tryptophan haltiges Futtermitteladditiv auf Fermentationsbrühebasis, welches folgende Merkmale aufweißt
a) einen Tryptophangehalt von mindestens 5 Gew.-% bis maximal 95 Gew.-%,
b) einen Wassergehalt von höchstens 5 Gew.-%, und
c) einen Biomassegehalt ensprechend mindestens 0,1 % der in der Fermentationsbrühe enthaltenen Biomasse, wobei die gegebenenfalls inaktivierte Biomasse aus erfindungsgemäßen coryneformen Bakterien gebildet wird.

Eine Mutante von Corynebacterium glutamicum mit der Bezeichnung DM1797, die den Aminosäureaustausch lysC T311I in der Aspartatkinase enthält, wurde am 28. Oktober 2004 bei der Deutschen Sammlung für Mikroorganismen und Zellkulturen (DSMZ, Braunschweig, Deutschland) als DSM 16833 hinterlegt.

Die erfindungsgemäße Mutante Corynebacterium glutamicum DM1808, die L-Phenylalanin an Position 111 der Aminosäuresequenz des Mqo-Polypeptids enthält, wurde am 24. November 2004 bei der Deutschen Sammlung für Mikroorganismen und Zellkulturen (DSMZ, Braunschweig, Deutschland) als DSM 16937 hinterlegt.

### Beispiele

### Beispiel 1

### Mutagenese des L-Lysin produzierenden Stammes DM1797

Der Corynebacterium glutamicum Stamm DM1797 wurde als Ausgangsstamm für die Mutagenese mit N-Methyl-N'-Nitro-N-Nitrosoguanidin (MNNG) eingesetzt. Der Stamm DM1797 ist eine Aminoethylcystein-resistente Mutante von Corynebacterium glutamicum ATCC13032 und unter der Bezeichnung DSM16833 bei der Deutschen Sammlung für Mikroorganismen und Zellkulturen (DSMZ, Braunschweig, Deutschland) hinterlegt.

Der Stamm DM1797 wurde in 10 ml LB-Bouillon (Merck, Darmstadt, Germany), die in einem 100 ml Erlenmeyerkolben enthalten waren, für 24 Stunden bei 33°C und 200 rpm auf einem Rotations-Schüttler vom Typ Certomat BS-1 (B. Braun Biotech International, Melsungen, Deutschland) kultiviert. Anschließend wurde die Kultur abzentrifugiert, das Sediment in 10 ml 0,9% NaCl-Lösung resuspendiert, die erhaltene Suspension erneut abzentrifugiert und das erhaltene Sediment in 10 ml 0,9% NaCl-Lösung aufgenommen. 5 ml dieser Zellsuspension wurden mit 400µg/ml MNNG für 15 Minuten bei 30°C und 200 rpm auf einem Schüttler (siehe oben) behandelt. Anschließend wurde der Mutageneseansatz abzentrifugiert und das Sediment in 10 ml 2% Na-Thiosulfat in 0,9% NaCl-Puffer (pH = 6,0) aufgenommen. Die Zellsuspension wurde anschließend im Verhältnis 1:1000, 1:10000 und 1:100000 mit 0,9% NaCl-Lösung verdünnt, und Aliquots auf Hirn-Herz-Agar (Merck, Darmstadt, Deutschland) ausplattiert. Auf diese Weise wurden ungefähr 2500 Mutanten isoliert.

### Beispiel 2

### Leistungstest der Mutanten des Stammes DM1797

Die in Beispiel 1 erhaltenen Mutanten wurden in einem zur Produktion von Lysin geeigneten Nährmedium kultiviert und der Lysingehalt im Kulturüberstand bestimmt.

Dazu wurden die Klone zunächst auf Hirn-Herz-Agarplatten (Merck, Darmstadt, Deutschland) für 24 Stunden bei 33°C vermehrt. Ausgehend von diesen Agarplattenkulturen wurde je eine Vorkultur angeimpft (10 ml Medium im 100 ml Erlenmeyerkolben). Als Medium für die Vorkultur wurde das Medium MM verwendet. Die Vorkultur wurde 24 Stunden bei 33°C und 240 rpm auf einem Schüttler inkubiert. Von dieser Vorkultur wurde eine Hauptkultur angeimpft, so dass die Anfangs-OD (660 nm) der Hauptkultur 0,1 OD betrug. Für die Hauptkultur wurde ebenfalls das Medium MM verwendet.

### Medium MM

| | |
|---|---|
| CSL | 5 g/l |
| MOPS | 20 g/l |
| Glucose (getrennt autoklaviert) | 50 g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| CaCO₃ | 25 g/l |

CSL (Corn Steep Liquor), MOPS (Morpholinopropansulfonsäure) und die Salzlösung wurden mit Ammoniakwasser auf pH 7 eingestellt und autoklaviert. Anschließend wurden die sterilen Substrat- und Vitaminlösungen sowie das trocken autoklavierte CaCO₃ zugesetzt.

Die Kultivierung erfolgte in Volumina von 10 ml, die in 100 ml Erlenmeyerkolben mit Schikanen enthalten waren. Die Temperatur betrug bei 33°C, die Umdrehungszahl war 250 rpm und die Luftfeuchte 80%.

Nach 48 Stunden wurde die optische Dichte (OD) bei einer Meßwellenlänge von 660 nm mit dem Biomek 1000 (Beckmann Instruments GmbH, München) bestimmt. Die gebildete Lysinmenge wurde mit einem Aminosäureanalysator der Firma Eppendorf-BioTronik (Hamburg, Deutschland) durch Ionenaustauschchromatographie und Nachsäulenderivatisierung mit Ninhydrindetektion bestimmt. Eine Mutante, die sich durch eine erhöhte Lysinbildung auszeichnete, wurde als DM1808 bezeichnet.

**Tabelle 1**

| Stamm | OD(660) | Lysin-HCl (g/l) |
|---|---|---|
| DM1797 | 12,1 | 4, 9 |
| DM1808 | 12,0 | 5,3 |

### Beispiel 3

### Sequenzierung des mqo-Gens der Mutante DM1808

Aus dem Klon DM1808 wurde mit der Methode von Eikmanns et al. (Microbiology 140: 1817 - 1828 (1994)) chromosomale DNA isoliert. Mit Hilfe der Polymerase-Kettenreaktion wurde ein DNA-Abschnitt amplifiziert, welcher das mqo-Gen trägt. Hierfür wurden folgende Oligonukleotide als Primer verwendet:
mqo-A1 (SEQ ID NO: 13):
   5' ggtgaaacttccgcgatact 3'
mqo-E1 (SEQ ID NO: 14):
   5' gtgtcgccta aatcacactg 3'

Die dargestellten Primer wurden von der Firma MWG Biotech (Ebersberg, Deutschland) synthetisiert. Sie ermöglichen die Amplifizierung eines ca. 2 kb langen DNA-Abschnittes, welcher das mqo-Gen trägt. Der Primer mqo-A1 bindet an die Region entsprechend Position 22 bis 41 des zu SEQ ID NO: 3 komplementären Stranges. Der Primer mqo-E1 bindet an die Region ensprechend Position 2002 bis 1983 des Stranges gemäß SEQ ID NO: 3.

Die PCR-Reaktion wurde mit der Phusion High Fidelity DNA Polymerase (New England Biolabs, Frankfurt, Deutschland) durchgeführt. Der Reaktionsansatz wurde nach Herstellerangaben angesetzt und enthielt bei 50 µl GesamtVolumen 10 µl des mitgelieferten 5 x Phusion HF Buffer, Desoxynucleosidtriphosphate in einer Konzentration von jeweils 200 pM, Primer in einer Konzentration von 0,5 µM, ungefähr 50 ng Template-DNA und 2 Units Phusion Polymerase. Durch Zugabe von H₂O wurde das Volumen auf 50 µl eingestellt.

Der PCR-Ansatz wurde zuerst einer einleitenden Denaturierung bei 98°C für 30 Sekunden unterzogen. Darauf folgten sich 35x wiederholend ein Denaturierungsschritt bei 98°C für 20 Sekunden, ein Schritt zum Binden der Primer an die vorgelegte DNA bei 60°C für 20 Sekunden und der Extensionsschritt zur Verlängerung der Primer bei 72°C für 60 Sekunden. Nach dem abschliessenden Extensionsschritt für 5 Minuten bei 72°C wurde der PCR-Ansatz einer Agarosegel-Elektrophorese (0,8% Agarose) unterworfen. Ein DNA-Fragment von ca. 2 kb Länge wurde identifiziert, aus dem Gel isoliert und unter Verwendung des QIAquick Gel Extraction Kit der Firma Qiagen, (Hilden, Deutschland) aufgereinigt.

Die Nukleotidsequenz des amplifizierten DNA-Fragmentes bzw. PCR-Produktes wurde von der Firma Agowa (Berlin, Deutschland) bestimmt. Die erhaltene Sequenz der Kodierregion des mqo-Allels ist in der SEQ ID NO: 5 dargestellt. Die sich mit Hilfe des Programmes Patentin ergebende Aminosäuresequenz des Proteins ist in der SEQ ID NO: 6 dargestellt.

Die Nukleotidsequenz der Kodierregion des mqo-Allels der Mutante DM1808 enthält an Position 332 die Nukleobase Thymin (Siehe SEQ ID NO: 5). Das Gen des Wildtyps (Siehe SEQ ID NO: 1) enthält an dieser Position die Nukleobase Cytosin. Diese Cytosin-Thymin Transition führt zu einem Aminosäureaustausch von Serin zu Phenylalanin an Position 111 der resultierenden Aminosäuresequenz. Diese Mutation wird im Folgenden als mqoS111F bezeichnet.

### Beispiel 4

### Konstruktion des Austauschvektors pK18mobsacB_mqoS111F

Mit Hilfe der Polymerase-Kettenreaktion wurde ein Teil der Kodierregion, das heisst ein sogenanntes internes Fragment bzw. interner Bereich, des mqo-Allels amplifiziert, der die Mutation mqoS111F trägt. Als Template wurde die in Beispiel 3 gewonnene chromosomale DNA verwendet. Folgende Oligonukleotide wurden als Primer für die PCR ausgewählt:
mqo-int1-bam (SEQ ID NO: 27):
   5' ctag-ggatcc-ccgaagaacgcaccgaggat 3'
mqo-int2-bam (SEQ ID NO: 28):
   5' ctag-ggatcc-ggcggatggacttgaacagg 3'

Sie wurden von der Firma MWG Biotech (Ebersberg, Deutschland) synthetisiert und ermöglichen die Amplifizierung eines ca. 1,05 kb langen DNA-Abschnittes der Kodierregion. Die Nukleotide 11 bis 30 des Primers mqoint1-bam binden an die Region entsprechend Position 362 bis 381 des zu SEQ ID NO:3 koplementären Stranges. Die Positionen 362 und 381 von SEQ ID NO:3 entsprechen den Positionen 13 und 32 in SEQ ID NO:1. Die Nukleotide 11 bis 30 des Primers mqo-int2-bam binden an die Region entsprechend Position 1385 bis 1366 des Stranges gemäß SEQ ID No. 3. Die Position 1385 und 1366 von SEQ ID NO:3 ensprechen den Positionen 1036 und 1017 von SEQ ID NO:1. Außerdem enthalten die Primer die Sequenzen für Schnittstellen der Restriktionsendonuklease BamHI, die in der oben dargestellten Nukleotidabfolge durch Unterstreichen markiert sind.

Die PCR-Reaktion wurde mit der Phusion High-Fidelity DNA Polymerase (New England Biolabs, Frankfurt, Deutschland) durchgeführt. Der Reaktionsansatz hatte die oben beschriebene Zusammensetzung. Die PCR wurde mit einer Ausnahme wie oben durchgeführt: der 72°C-Extensionsschritt in der 35-fachen Wiederholung wurde jeweils nur für 30 Sekunden durchgeführt.

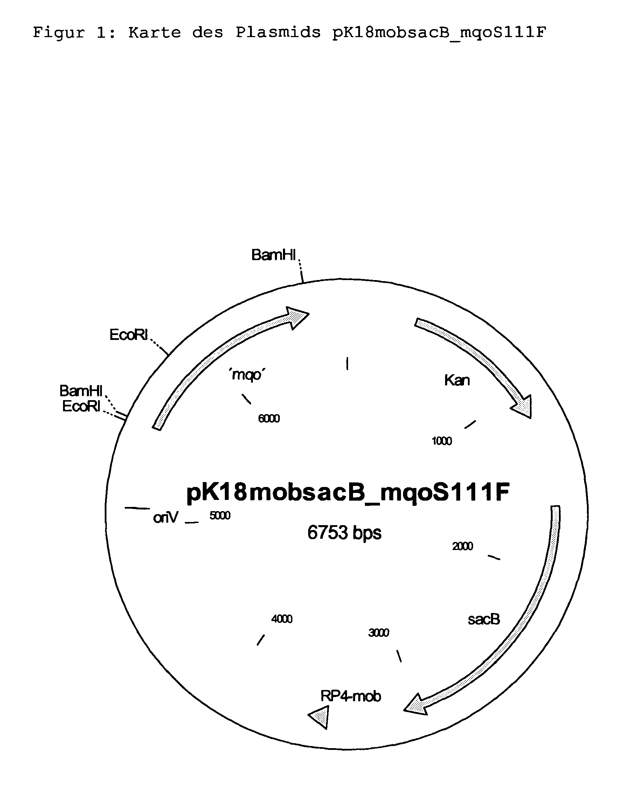

Das ca. 1,05 kb lange Amplifikat wurde mit der Restriktionsendonuklease BamHI behandelt und durch Elektrophorese in einem 0,8%igen Agarosegel identifiziert. Es wurde anschließend aus dem Gel isoliert und mit dem QIAquick Gel Extraction Kit der Firma Qiagen aufgereinigt. Das dergestalt gereinigte DNA-Fragment enthält die beschrieben mqoS111F-Mutation und besitzt BamHI kompatible Enden (mqoS111F-Fragment bzw. 'mqo' in Figur 1). Es wurde anschließend in den von Schäfer et al. (Gene, 145, 69-73 (1994) beschriebenen, mobilisierbaren Vektor pK18mobsacB eingebaut, um einen Allel- beziehungsweise Mutationsaustausch zu ermöglichen. Hierzu wurde pK18mobsacB mit dem Restriktionsenzym BamHI verdaut und die Enden mit alkalischer Phosphatase (Alkaline Phosphatase, Boehringer Mannheim, Deutschland) dephosphoryliert. Der so vorbereitete Vektor wurde mit dem mqoS111F-Fragment gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al.,Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2nd Ed. Cold Spring Habor, New York, 1989), der mit 25 mg/l Kanamycin supplementiert worden war.

Plasmid-DNA wurde aus einer Transformante mit Hilfe des QIAprep Spin Miniprep Kit der Firma Qiagen isoliert und durch Restriktionsspaltung jeweils einmal mit dem Enzym BamHI und einmal mit dem Enzym EcoRI und anschließender Agarosegel-Elektrophorese überprüft. Das Plasmid wurde pK18mobsacB_mqoS111F genannt und ist in Figur 1 dargestellt.

### Beispiel 5

### Einbau der Mutation mqoS111F in den Stamm DM1797

Der in Beispiel 4 beschriebene Vektor pK18mobsacB_mqoS111F wurde nach dem Protokoll von Schäfer et al. (Journal of Microbiology 172: 1663-1666 (1990)) in den C. glutamicum Stamm DM1797 durch Konjugation transferiert. Der Vektor kann in DM1797 nicht selbständig replizieren und bleibt nur dann in der Zelle erhalten, wenn er als Folge eines Rekombinationsereignisses im Chromosom integriert vorliegt. Die Selektion von Transkonjuganten, d. h. von Klonen mit integriertem pK18mobsacB_mqoS111F erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_mqoS111F enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, die die Synthese des für C. glutamicum toxischen Produktes Levan katalysiert. Auf mit Sucrose supplementiertem LB-Agar wachsen daher nur solche Klone, bei denen das integrierte pK18mobsacB_mqoS111F als Folge eines zweiten Rekombinationsereignisses exzisiert hat. In Abhängigkeit von der Lage des zweiten Rekombinationsereignisses in bezug auf den Mutationsort findet bei der Exzision der Allelaustausch bzw. der Einbau der Mutation statt oder es verbleibt die ursprüngliche Kopie im Chromosom des Wirtes.

Anschließend wurde ein Klon gesucht, bei dem der gewünschte Austausch, d. h. der Einbau der Mutation mqoS111F, erfolgt war. Hierzu wurde von 10 Klonen mit dem Phänotyp "Wachstum in Gegenwart von Sucrose" und "Nicht-Wachstum in Gegenwart von Kanamycin" die Sequenz des mqo-Gens bestimmt. Auf diese Weise wurde ein Klon identifiziert, der die Mutation mqoS111F trägt. Dieser Stamm wurde als C. glutamicum DM1797_mqoS111F bezeichnet.

### Beispiel 6

Vergleich der Leistung des Stammes DM1797_mqoS111F mit der des Ausgangsstammes DM1797

Der Leistungtest wurde wie in Beispiel 2 beschrieben durchgeführt. Der Stamm DM1797_mqoS111F zeigte im Vergleich zu DM1797 deutlich erhöhte Lysinausscheidung ähnlich wie DM1808 (Siehe Tabelle 1).

Kurze Beschreibung der Figur:
Figur 1: Karte des Plasmids pK18mobsacB_mqoS111F

Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.
- Kan:: Kanamycin Resistenz-Gen
- BamHI:: Schnittstelle des Restriktionsenzyms EcoRI
- EcoRI:: Schnittstelle des Restriktionsenzyms EcoRI
- 'mqo':: kloniertes DNA-Fragment enthaltend einen internen Bereich des Allels mqoS111F
- sacB:: sacB-Gen
- RP4-mob:: mob-Region mit dem Replikationsursprung für den Transfer (oriT)
- oriV:: Replikationsursprung V

## Patentansprüche

1. Isolierte Mutanten coryneformer Bakterien, welche ein Gen enthalten, ausgewählt aus einem der folgenden a) bis c):
a) ein Gen, das für ein Polypeptid mit Malat-Chinon-Oxidoreduktase Aktivität kodiert, wobei das Polypeptid die Aminosäuresequenz SEQ ID NO:2 umfasst, bei der an Position 111 der Aminosäuresequenz von SEQ ID NO:2 L-Phenylalanin enthalten ist und gegebenenfalls L-Serin an der Position 201 von SEQ ID NO: 2 enthalten ist;
b) ein Gen, das für ein Polypeptid mit Malat-Chinon-Oxidoreduktase Aktivität kodiert, wobei das kodierte Polypeptid eine Aminosäuresequenz umfasst, die mindestens 98% identisch ist mit der Aminosäuresequenz von SEQ ID NO: 6, wobei in der Aminosäuresequenz an der Position, die der Position 111 der Aminosäuresequenz SEQ ID NO: 6 entspricht, L-Phenylalanin enthalten ist;
c) ein Gen, das für ein Protein mit Malat-Chinon Oxidoreduktase Aktivität kodiert, wobei das kodierte Protein eine Aminosäuresequenz gemäß SEQ ID NO: 6 einschließlich eines bis 5 konservativer Aminosäureaustausch(e) umfasst, wobei in der Aminosäuresequenz an der Position, die der Position 111 der Aminosäuresequenz SEQ ID NO: 6 entspricht, L-Phenylalanin enthalten ist;
wobei die Aminosäuresekretion dieser Mutanten im Vergleich zum nicht mutagenisierten Ausgangsstamm mit der Aminosäuresequenz gemäß SEQ ID NO: 2 um mindestens 0,5% erhöht ist.

2. Mutanten coryneformer Bakterien gemäß Anspruch 1, **dadurch gekennzeichnet, dass** es sich bei den coryneformen Bakterien um ein Bakterium ausgewählt aus der Gruppe Corynebacterium efficiens, Corynebacterium glutamicum, Corynebacterium thermoaminogenes und Corynebacterium aminogenes handelt.

3. Mutanten coryneformer Bakterien gemäß Anspruch 2, **dadurch gekennzeichnet, dass** es sich um Corynebacterium glutamicum handelt.

4. Mutanten coryneformer Bakterien gemäß Anspruch 1, **dadurch gekennzeichnet, dass** es sich um L-Lysin oder um L-Tryptophan oder um L-Prolin ausscheidende Bakterien handelt.

5. Mutanten coryneformer Bakterien gemäß Anspruch 1, **dadurch gekennzeichnet, dass** das Gen die Nukleotidsequenz von SEQ ID NO: 5 umfasst.

6. Ein isoliertes Polynukleotid ausgewählt aus einem der folgenden a) oder b)
a) ein Polynukleotid, das für ein Polypeptid mit Malat-Chinon-Oxidoreduktase Enzymaktivität und der Aminosäuresequenz SEQ ID NO:2 kodiert, welches an Position 111 der Aminosäuresequenz von SEQ ID NO: 2 L-Phenylalanin enthält und gegebenenfalls L-Serin an der Position 201 von SEQ ID NO: 2 besitzt.
b) ein Polynukleotid, wobei das kodierte Protein eine Aminosäuresequenz gemäß SEQ ID NO: 6 einschließlich eines bis 5 konservativer Aminosäureaustausch(e) umfasst, wobei in der Aminosäuresequenz an der Position, die der Position 111 der Aminosäuresequenz von SEQ ID NO: 6 entspricht, L-Phenylalanin enthalten ist,
wobei diese Polynukleotide die Aminosäuresekretion entsprechender Mutanten im Vergleich zum nicht mutagenisierten Ausgangsstamm mit der Aminosäuresequenz gemäß SEQ ID NO: 2 um mindestens 0,5 % erhöhen.

7. Isoliertes Polynukleotid gemäß Anspruch 6, **dadurch gekennzeichnet, dass** das kodierte Polypeptid mit der Aminosäuresequenz SEQ ID NO: 2 eine Länge von 500 Aminoäuren umfasst, und an Position 111 der Aminosäuresequenz von SEQ ID NO:2 L-Phenylalanin enthalten ist.

8. Isoliertes Polynukleotid gemäß Anspruch 7, **dadurch gekennzeichnet, dass** das kodierte Polypeptid die Aminosäuresequenz von SEQ ID NO: 6 umfasst.

9. Isoliertes Polynukleotid gemäß Anspruch 8, **dadurch gekennzeichnet, dass** die Nukleotidsequenz SEQ ID NO: 5 umfasst.

10. Ein isoliertes Polynukleotid, welches ein Nukleinsäure-Molekül umfasst, das mindestens für ein Leseraster mit einer Aminosäuresequenz entsprechend Position 95 bis 127 von SEQ ID NO: 2 kodiert, wobei an der Position entsprechend Position 111 von SEQ ID NO: 2 L-Phenylalanin enthalten ist, zur Verwendung zur Herstellung von coryneformen Bakterien, die eine im Vergleich zum nicht mutagenisierten Ausgangsstamm mit der Aminosäuresequenz gemäß SEQ ID NO: 2 um mindestens 0,5% erhöhte Aminosäuresektretion aufweisen.

11. Isoliertes Polynukleotid gemäß Anspruch 10, **dadurch gekennzeichnet, dass** es mindestens die Nukleotidsequenz entsprechend Position 283 bis 381 von SEQ ID NO: 5 umfasst.

12. Verfahren zur Herstellung eines rekombinanten coryneformen Bakteriums, **dadurch gekennzeichnet, dass** man
a) ein isoliertes Polynukleotid gemäß Anspruch 6 bis 9 oder gemäß Anspruch 10 bis 11 in ein coryneformes Bakterium überführt,
b) das im Chromosom des coryneformen Bakteriums vorhandene Malat-Chinon-Oxidoreduktase Gen, das für eine
Aminosäuresequenz mit L-Serin an Position 111 der Aminosäuresequenz SEQ ID NO: 2 kodiert, gegen das Polynukleotid aus a) austauscht, das für eine Aminosäuresequenz kodiert, die L-Phenylalanin an der Position 111 von SEQ ID NO: 2 und gegebenenfalls L-Serin an der Position 201 von SEQ ID NO: 2 besitzt, und
c) das gemäß Schritt a) und b) erhaltene coryneforme Bakterium vermehrt.

13. Ein Vektor, der das isolierte Polynukleotid gemäß Anspruch 6 bis 90 oder 10 bis 11 enthält.

14. Ein rekombinanter Mikroorganismus, der den Vektor gemäß Anspruch 13 enthält, **dadurch gekennzeichnet, dass** es sich um ein coryneformes Bakterium handelt.

15. Rekombinanter Mikroorganismus gemäß Anspruch 14, **dadurch gekennzeichnet, dass** es sich bei dem coryneformen Bakterium um die Gattung Corynebacterium handelt.

16. Rekombinanter Mikroorganismus gemäß Anspruch 15, **dadurch gekennzeichnet, dass** es sich bei dem Bakterium der Gattung Corynebacterium um die Art Corynebacterium glutamicum handelt.

17. Verfahren zur Herstellung einer L-Aminosäure **dadurch gekennzeichnet, dass** man
a) ein isoliertes coryneformes Bakterium in einem geeignetem Medium fermentiert, wobei das Bakterium ein für ein Polypeptid mit Malat-Chinon-Oxidoreduktase Enzymaktivität kodierendes Gen enthält, wobei in den Aminosäuresequenzen des Polypeptids das L-Serin an der Position 111 oder der entsprechenden Position durch L-Phenylalanin ersetzt ist und wobei es sich bei dem Gen um ein Polynukleotid nach einem der Ansprüche 6 bis 11 handelt, und
b) die L-Aminosäure in der Fermentationsbrühe oder in den Zellen des Bakteriums anreichert.

18. Verfahren gemäß Anspruch 17, **dadurch gekennzeichnet, dass** es sich bei dem isolierten coryneformen Bakterium um eine Mutante gemäß Anspruch 1 bis 6 handelt.

19. Verfahren nach Anspruch 17, **dadurch gekennzeichnet, dass** man die L-Aminosäure isoliert oder sammelt.

20. Verfahren nach Anspruch 19, **dadurch gekennzeichnet, dass** man die L-Aminosäure reinigt.

21. Verfahren nach Anspruch 17, **dadurch gekennzeichnet, dass** man die L-Aminosäure gemeinsam mit Bestandteilen aus der Fermentationsbrühe und/oder der Biomasse (> 0 bis 100%) isoliert oder sammelt.

22. Verfahren nach Anspruch 17, **dadurch gekennzeichnet, dass** man
a) aus der in Schritt b) von Anspruch 17 erhaltenen Fermentationsbrühe die gebildete Biomasse in einer Menge von 0 bis 100% entfernt, und
b) aus der in Schritt a) erhaltenen Brühe ein im wesentlichen trockenes und geformtes Produkt durch eine Methode ausgewählt aus der Gruppe Granulation, Kompaktierung, Sprühtrocknung und Extrusion herstellt.

23. Verfahren nach Anspruch 22, **dadurch gekennzeichnet, dass** man der Fermentationsbrühe vor oder nach Schritt a) eine Säure ausgewählt aus der Gruppe Schwefelsäure, Salzsäure und Phosphorsäure hinzufügt.

24. Verfahren nach Anspruch 22, **dadurch gekennzeichnet, dass** man aus der vor oder nach in Schritt a) erhaltenen Brühe Wasser entfernt.

25. Verfahren gemäß Anspruch 22, **dadurch gekennzeichnet, dass** man das in oder während Schritt b) erhaltene geformte Produkt mit einem Öl besprüht.

## Claims

1. Isolated mutants of coryneform bacteria which contain a gene selected from one of the following a) to c) :
a) a gene which codes for a polypeptide with malate quinone oxidoreductase activity, wherein the polypeptide comprises the amino acid sequence SEQ ID No: 2, wherein L-phenylalanine is contained at position 111 of the amino acid sequence of SEQ ID No:2 and optionally L-serine is contained at position 201 of SEQ ID No: 2;
b) a gene which codes for a polypeptide with malate quinone oxidoreductase activity, wherein the encoded polypeptide comprises an amino acid sequence which is at least 98% identical with the amino acid sequence of SEQ ID No: 6, wherein L-phenylalanine is contained in the amino acid sequence at the position which corresponds to position 111 of the amino acid sequence of SEQ ID No: 6;
c) a gene which codes for a polypeptide with malate quinone oxidoreductase activity, wherein the encoded protein comprises an amino acid sequence according to SEQ ID No: 6 including one to 5 conservative amino acid substitution(s), wherein L-phenylalanine is contained in the amino acid sequence at the position which corresponds to position 111 of the amino acid sequence of SEQ ID No: 6;
wherein the amino acid secretion of these mutants is increased by at least 0.5% in comparison to the non-mutagenized starting strain with the amino acid sequence according to SEQ ID NO.2.

2. Mutants of coryneform bacteria according to Claim 1, **characterized in that** the coryneform bacteria is a bacterium selected from the group Corynebacterium efficiens, Corynebacterium glutamicum, Corynebacterium thermoaminogenes and Corynebacterium aminogenes.

3. Mutants of coryneform bacteria according to Claim 2, **characterized in that** it is Corynebacterium glutamicum.

4. Mutants of coryneform bacteria according to Claim 1, **characterized in that** they are L-lysine or L-tryptophan or L-proline excreting bacteria.

5. Mutants of coryneform bacteria according to Claim 1, **characterized in that** the gene comprises the nucleotide sequence of SEQ ID No: 5.

6. Isolated polynucleotide selected from one of the following a) or b)
a) a polynucleotide which codes for a polypeptide with malate quinone oxidoreductase enzyme activity and the amino acid sequence SEQ ID No: 2, which contains L-phenylalanine at position 111 of the amino acid sequence of SEQ ID No: 2 and optionally possesses L-serine at position 201 of SEQ ID No: 2.
b) a polynucleotide, wherein the encoded protein comprises an amino acid sequence according to SEQ ID No: 6 including one to 5 conservative amino acid substitution(s), wherein L-phenylalanine is contained in the amino acid sequence at the position which corresponds to position 111 of the amino acid sequence of SEQ ID No: 6;
wherein these polynucleotides increase the amino acid secretion of corresponding mutants by at least 0.5% in comparison to the non-mutagenized starting strain with the amino acid sequence according to SEQ ID NO.2.

7. Isolated polynucleotide according to Claim 6, **characterized in that** the encoded polypeptide with the amino acid sequence SEQ ID No: 2 comprises a length of 500 amino acids, and L-phenylalanine is contained at position 111 of the amino acid sequence of SEQ ID No: 2.

8. Isolated polynucleotide according to Claim 7, **characterized in that** the encoded polypeptide comprises the amino acid sequence of SEQ ID No: 6.

9. Isolated polynucleotide according to Claim 8, **characterized in that** the nucleotide sequence comprises SEQ ID No: 5.

10. Isolated polynucleotide which comprises a nucleic acid molecule which at least codes for a reading frame with an amino acid sequence corresponding to position 95 to 127 of SEQ ID No: 2, wherein L-phenylalanine is contained at the position corresponding to position 111 of SEQ ID No: 2, for use for the production of coryneform bacteria which exhibit an amino acid secretion increased by at least 0.5% in comparison to the non-mutagenized starting strain with the amino acid sequence according to SEQ ID NO.2.

11. Isolated polynucleotide according to Claim 10, **characterized in that** it at least comprises the nucleotide sequence corresponding to position 283 to 381 of SEQ ID No: 5.

12. Method for the production of a recombinant coryneform bacterium, **characterized in that**
a) an isolated polynucleotide according to Claim 6 to 9 or according to Claim 10 to 11 is transferred into a coryneform bacterium,
b) the malate quinone oxidoreductase gene present in the chromosome of the coryneform bacterium, which codes for an amino acid sequence with L-serine at position 111 of the amino acid sequence SEQ ID No: 2, is replaced with the polynucleotide from a), which has L-phenylalanine at position 111 of SEQ ID No: 2 and optionally L-serine at position 201 of SEQ ID No: 2, and
c) the coryneform bacterium obtained according to steps a) and b) is propagated.

13. Vector which contains the isolated polynucleotide according to Claim 6 to 9 or 10 to 11.

14. Recombinant microorganism which contains the vector according to Claim 13, **characterized in that** it is a coryneform bacterium.

15. Recombinant microorganism according to Claim 14, **characterized in that** the coryneform bacterium is of the genus Corynebacterium.

16. Recombinant microorganism according to Claim 15, **characterized in that** the bacterium of the genus Corynebacterium is of the species Corynebacterium glutamicum.

17. Process for the production of an L-amino acid, **characterized in that**
a) an isolated coryneform bacterium is fermented in a suitable medium, wherein the bacterium contains a gene coding for a polypeptide with malate quinone oxidoreductase enzyme activity, wherein the L-serine at position 111 or the corresponding position in the amino acid sequences of the polypeptide is replaced by L-phenylalanine and wherein the gene is a polynucleotide according to one of Claims 6 to 11, and
b) the L-amino acid is enriched in the fermentation broth or in the cells of the bacterium.

18. Process according to Claim 17, **characterized in that** the isolated coryneform bacterium is a mutant according to Claims 1 to 6.

19. Process according to Claim 17, **characterized in that** the L-amino acid is isolated or collected.

20. Process according to Claim 19, **characterized in that** the L-amino acid is purified.

21. Process according to Claim 17, **characterized in that** the L-amino acid is isolated or collected together with constituents from the fermentation broth and/or the biomass (>0 to 100%) .

22. Process according to Claim 17, **characterized in that**
a) from 0 to 100% of the biomass formed is removed from the fermentation broth obtained in step b) of Claim 17, and
b) an essentially dry and formed product is produced by a method selected from the group granulation, compaction, spray drying and extrusion from the broth produced in step a).

23. Process according to Claim 22, **characterized in that** an acid selected from the group sulphuric acid, hydrochloric acid and phosphoric acid is added to the fermentation broth before or after step a).

24. Process according to Claim 22, **characterized in that** water is removed from the broth obtained before or after step a) .

25. Process according to Claim 22, **characterized in that** the formed product obtained in or during step b) is sprayed with an oil.

## Revendications

1. Mutants isolés de bactéries corynéformes, qui contiennent un gène choisi parmi un des gènes a) à c) ci-après .
a) un gène qui code pour un polypeptide ayant une activité de malate-quinone-oxydoréductase, le polypeptide contenant la séquence d'acides aminés SEQ ID NO:2, une L-phénylalanine étant présente en position 111 de la séquence d'acides aminés de SEQ ID NO:2 et une L-sérine étant éventuellement présente en position 201 de SEQ ID NO:2 ;
b) un gène qui code pour un polypeptide ayant une activité de malate-quinone-oxydoréductase, le polypeptide codé comprenant une séquence d'acides aminés qui a une identité d'au moins 98 % avec la séquence d'acides aminés de SEQ ID NO:6, une L-phénylalanine étant présente dans la séquence d'acides aminés sur la position qui correspond à la position 111 de la séquence d'acides aminés SEQ ID NO:6 ;
c) un gène qui code pour une protéine ayant une activité de malate-quinone-oxydoréductase, la protéine codée comprenant une séquence d'acides aminés selon SEQ ID NO:6, y compris 1 à 5 échanges d'acides aminés conservateurs, une L-phénylalanine étant présente dans la séquence d'acides aminés sur la position qui correspond à la position 111 de la séquence d'acides aminés SEQ ID NO:6 ;
la sécrétion d'acides aminés de ces mutants étant augmentée d'au moins 0,5 % par comparaison avec la souche de départ non mutagénisée ayant la séquence d'acides aminés selon SEQ ID NO:2.

2. Mutants de bactéries corynéformes selon la revendication 1, **caractérisés en ce que**, pour ce qui concerne les bactéries corynéformes, il s'agit d'une bactérie choisie dans le groupe *Corynebacterium efficiens, Corynebacterium glutamicum, Corynebacterium thermoaminogenes* et *Corynebacterium aminogenes.*

3. Mutants de bactéries corynéformes selon la revendication 2, **caractérisés en ce qu'**il s'agit de *Corynebacterium glutamicum.*

4. Mutants de bactéries corynéformes selon la revendication 1, **caractérisés en ce qu'**il s'agit de bactéries qui sécrètent de la L-lysine ou du L-tryptophane ou de la L-proline.

5. Mutants de bactéries corynéformes selon la revendication 1, **caractérisés en ce que** le gène comprend la séquence nucléotidique de SEQ ID NO:5.

6. Polynucléotide isolé choisi parmi un des polynucléotides a) ou b) ci-après :
a) un polynucléotide qui code pour un polypeptide ayant l'activité enzymatique de la malate-quinone-oxydoréductase et ayant la séquence d'acides aminés SEQ ID NO:2, qui en position 111 de la séquence d'acides aminés de SEQ ID NO:2 possède une L-phénylalanine et possède éventuellement une L-sérine en position 201 de SEQ ID NO:2,
b) un polynucléotide, la protéine codée comprenant une séquence d'acides aminés selon SEQ ID NO:6, y compris un ou plusieurs échanges d'acides aminés conservateurs, une L-phénylalanine étant présente dans la séquence d'acides aminés sur la position qui correspond à la position 111 de la séquence d'acides aminés de SEQ ID NO:6,
ces polynucléotides augmentant d'au moins 0,5 % la sécrétion d'acides aminés de mutants correspondants, par comparaison avec la souche de départ non mutagénisée ayant la séquence d'acides aminés selon SEQ ID NO:2.

7. Polynucléotide isolé selon la revendication 6, **caractérisé en ce que** le polypeptide codé ayant la séquence d'acides aminés SEQ ID NO:2 a une longueur de 500 acides aminés, et qu'une L-phénylalanine est présente en position 111 de la séquence d'acides aminés de SEQ ID NO:2.

8. Polynucléotide isolé selon la revendication 7, **caractérisé en ce que** le polypeptide codé comprend la séquence d'acides aminés de SEQ ID NO:6.

9. Polynucléotide isolé selon la revendication 8, **caractérisé en ce que** la séquence nucléotidique comprend SEQ ID NO:5.

10. Polynucléotide isolé, qui comprend une molécule d'acide nucléique qui code pour au moins un cadre de lecture ayant une séquence d'acides aminés correspondant aux positions 95 à 127 de SEQ ID NO:2, une L-phénylalanine étant présente sur la position correspondant à la position 111 de SEQ ID NO:2, pour une utilisation dans le but de fabriquer des bactéries corynéformes qui présentent une sécrétion d'acides aminés augmentée d'au moins 0,5 % par comparaison avec la souche de départ non mutagénisée ayant la séquence d'acides aminés selon SEQ ID NO:2.

11. Polynucléotide isolé selon la revendication 10, **caractérisé en ce qu'**il comprend au moins une séquence nucléotidique correspondant aux positions 283 à 381 de SEQ ID NO:5.

12. Procédé de fabrication d'une bactérie corynéforme recombinante, **caractérisé en ce que**
a) on transfère dans une bactérie corynéforme un polynucléotide selon les revendications 6 à 9 ou selon les revendications 10 à 11,
b) on remplace le gène de la malate-quinone-oxydoréductase présent dans le chromosome de la bactérie corynéforme, et qui code pour une séquence d'acides aminés ayant une L-sérine en position 111 de la séquence d'acides aminés SEQ ID NO:2, par le polynucléotide de a), qui code pour une séquence d'acides aminés qui possède une L-phénylalanine en position 111 de SEQ ID NO:2 et éventuellement une L-sérine en position 201 de SEQ ID NO:2, et
c) on multiplie la bactérie corynéforme obtenue selon les étapes a) et b).

13. Vecteur qui contient le polynucléotide isolé selon les revendications 6 à 9 ou 10 à 11.

14. Microorganisme recombinant qui contient le vecteur selon la revendication 13, **caractérisé en ce qu'**il s'agit d'une bactérie corynéforme.

15. Microorganisme recombinant selon la revendication 14, **caractérisé en ce que**, pour ce qui concerne la bactérie corynéforme, il s'agit du genre *Corynebacterium.*

16. Microorganisme recombinant selon la revendication 15, **caractérisé en ce que**, pour ce qui concerne la bactérie du genre *Corynebacterium,* il s'agit de l'espèce *Corynebacterium glutamicum.*

17. Procédé de fabrication d'un acide L-aminé, **caractérisé en ce que**
a) on fermente une bactérie corynéforme isolée dans un milieu approprié, la bactérie contenant un gène codant pour un polypeptide ayant l'activité enzymatique de la malate-quinone-oxydoréductase, la L-sérine en position 111 ou sur la position correspondante étant, dans les séquences d'acides aminés du polypeptide, remplacée par une L-phénylalanine, et dans laquelle, pour ce qui concerne le gène, il s'agit d'un polynucléotide selon l'une des revendications 6 à 11, et
b) on enrichit l'acide L-aminé dans le bouillon de fermentation ou dans les cellules de la bactérie.

18. Procédé selon la revendication 17, **caractérisé en ce que**, pour ce qui concerne la bactérie corynéforme isolée, il s'agit d'un mutant selon les revendications 1 à 6.

19. Procédé selon la revendication 17, **caractérisé en ce qu'**on isole ou recueille l'acide L-aminé.

20. Procédé selon la revendication 19, **caractérisé en ce qu'**on purifie l'acide L-aminé.

21. Procédé selon la revendication 17, **caractérisé en ce qu'**on isole ou recueille l'acide L-aminé en même temps que des constituants provenant du bouillon de fermentation et/ou de la biomasse (> 0 à 100 %).

22. Procédé selon la revendication 17, **caractérisé en ce que**
a) on élimine du bouillon de fermentation obtenu dans l'étape b) de la revendication 17 la biomasse formée, en une quantité de 0 à 100 %, et
b) à partir du bouillon obtenu dans l'étape a), on fabrique un produit, pour l'essentiel sec et façonné, par une méthode choisie dans le groupe consistant en la granulation, le compactage, le séchage par atomisation et l'extrusion.

23. Procédé selon la revendication 22, **caractérisé en ce qu'**on ajoute au bouillon de fermentation, avant ou après l'étape a), un acide choisi dans le groupe consistant en l'acide sulfurique, l'acide chlorhydrique et l'acide phosphorique.

24. Procédé selon la revendication 22, **caractérisé en ce qu'**on élimine l'eau du bouillon obtenu avant ou après l'étape a).

25. Procédé selon la revendication 22, **caractérisé en ce qu'**on pulvérise une huile sur le produit façonné obtenu dans ou pendant l'étape b).
